# EUROPEAN PATENT SPECIFICATION

(11) **EP 2 677 031 B1**
(45) Date of publication and mention of the grant of the patent: **28.09.2016**
(21) Application number: 13185458.0
(22) Date of filing: 05.09.2011
(51) Int. Cl.: C12N 15/113, A61K 31/00, A61K 31/713, A61P 3/10

(54) **GRK5 Kinases as targets for anti-diabetic therapy**
GRK5 Kinasen als Ziele für antidiabetische Therapie
Kinases GRK5 en tant que cibles pour une thérapie antidiabétique

(30) Priority: 03.09.2010 EP 10075383; 08.09.2010 US 344668 P
(43) Date of publication of application: 25.12.2013
(62) Divisional of application: 11760402.5
(73) Proprietor: Max-Planck-Gesellschaft zur Förderung der Wissenschaften e.V., 80539 München (DE)
(72) Inventor: Falcenberg, Mathias, 82152 Martinsried (DE); Ullrich, Axel, 80331 Munich (DE)
(74) Representative: Arth, Hans-Lothar

(56) References cited:
- WO-A1-2005/113799
- MOKHTARI DARIUSH ET AL: "Potential utility of small tyrosine kinase inhibitors in the treatment of diabetes.", CLINICAL SCIENCE (LONDON, ENGLAND : 1979) FEB 2010 LNKD- PUBMED:19886867, vol. 118, no. 4, February 2010 (2010-02), pages 241-247, XP002663612, ISSN: 1470-8736
- BILLEMONT B ET AL: "Blood glucose levels in patients with metastatic renal cell carcinoma treated with sunitinib.", BRITISH JOURNAL OF CANCER 4 NOV 2008 LNKD- PUBMED:18841151, vol. 99, no. 9, 4 November 2008 (2008-11-04), pages 1380-1382, XP002621250, ISSN: 1532-1827
- SORRIENTO D ET AL: "Kinase independent inhibition of NF.kappa.B transcriptional activity by GRK5 through I.kappa.B.alpha stabilization", NATURE PRECEDINGS , 29 September 2007 (2007-09-29), pages 1-29, XP002544007, ISSN: 1756-0357 Retrieved from the Internet: URL:http://precedings.nature.com/documents /1196/version/1/files/npre20071196-1.pdf [retrieved on 2013-10-29]
- ISAO USUI ET AL: "GRK2 is an endogenous protein inhibitor of the insulin signaling pathway for glucose transport stimulation", THE EMBO JOURNAL, vol. 23, no. 14, 21 July 2004 (2004-07-21) , pages 2821-2829, XP055086130, ISSN: 0261-4189, DOI: 10.1038/sj.emboj.7600297
- LI WANG ET AL: "GRK5 ablation contributes to insulin resistance", BIOCHEMICAL AND BIOPHYSICAL RESEARCH COMMUNICATIONS, vol. 429, no. 1-2, 1 December 2012 (2012-12-01), pages 99-104, XP055086121, ISSN: 0006-291X, DOI: 10.1016/j.bbrc.2012.10.077

## Description

The present invention is related to compound capable of modulating the activity and/or expression of certain protein kinases thereby enhancing the expression and/or release of insulin. The invention is further related to methods of identifying said compounds for the treatment of metabolic diseases. The invention is further related to methods of treatment of metabolic diseases, particularly diabetes mellitus type 2.

Diabetes as a leading cause of death in developed countries is a metabolic condition characterized by high blood sugar levels. There are two main types of diabetes: type 1, resulting from insufficient insulin production of the pancreas beta cells, which requires the person to inject insulin; and type 2, resulting from insensitivity of peripheral tissues (such skeletal muscle, liver or adipose tissue) insulin release alterations, and relative insulin deficiency. Diabetes mellitus type 2 is often acquired and accompanied by obesity; it can be treated in first hand by reducing weight, diet and exercise. Type 1 diabetes is a genetic or autoimmune disease; the only effective therapy to date is the supply of exogenous insulin. This therapy does not cure diabetes; the person needs continuous supply of insulin.

The decreased insulin sensitivity of peripheral tissues in type 2 diabetes which accounts for 90% of all cases of the disease is initially compensated by an increased release of insulin by the beta cells of the pancreas. At a certain stage of the disease, the pancreas cannot maintain the increases release of insulin anymore. As disease progresses, drugs which are currently available and elevate insulin release have lead to beta-cell damage and loss of insulin production.

A number of diseases, including cancer, diabetes and inflammation are linked to perturbation of protein kinase mediated cell signaling pathways. For some time, a new class of multiple kinase drugs has been undergoing clinical trials. Some have been approved for various applications, mostly for the treatment of cancer. The targets of these multiple kinase inhibitors like Imanitib or Sunitinib interact at all stages of signal transduction: from the receptor tyrosine kinases which initiate intracellular signaling to second-messenger generators and kinases involved in signaling cascades and finally to those kinases which regulate the cell cycle governing cellular fate.

Several publications have shown the effect of kinase-inhibitors like Sunitinib (Sutent^{®}) and Imatinib (Gleevec^{®}) on diabetes during a period of treatment which leads to a remission of diabetes type 1 or 2 in patients. However, only few kinases could be identified that affect the insulin release or sensitivity specifically to develop a more specific treatment strategy.

In prior art (Mokhtari et al., 2010, Clinical Science 118, 241-247 and Billemont et al., 2008, Brit J Cancer 99, 1380 - 1382) it was described that the multiple kinase inhibitor sunitinib has anti-diabetic effects (e.g. caused by increased insulin sensitivity) and is therefore suitable for use in the treatment of diabetes. The crucial target of sunitinib was not described.

The objective of the present invention is to provide targets for the treatment of metabolic diseases such as diabetes, compounds which are useful for raising the blood insulin level by enhancing the insulin release of the pancreatic beta cells and methods for identifying such compounds. This goal is achieved by the compounds which bind to regulate the protein kinase according to claim 1 as well as by the methods for identifying such compounds. Further advantageous embodiments, aspects and details of the invention are evident from the claims, the description, the examples and the figures.

### Short description

The invention refers particularly to a modulator for the inhibition of the activity of the protein kinase GRK5 for use in the treatment of a disease of the carbohydrate metabolism, more preferred of diabetes, more preferred of diabetes mellitus type 2, and most preferred for the up-regulation of insulin production and/or release of insulin, wherein the modulator is a small molecule, an siRNA molecule, or an antisense oligonucleotide and wherein the modulator is not sunitinib. The invention refers further to a modulator for the inactivation, degradation, downregulation, intercalation of at least one nucleic acid encoding GRK5 for use in the treatment of a disease of the carbohydrate metabolism, more preferred of diabetes, more preferred of diabetes mellitus type 2, and most preferred for the up-regulation of insulin production and/or release of insulin, wherein the modulator is a small molecule, an siRNA molecule, or an antisense oligonucleotide and wherein the modulator is not sunitinib.

The said modulator can be chosen from the group comprising a small molecule, a siRNA molecule, , an antisense oligonucleotide.

The invention refers further to a pharmaceutical composition comprising said modulator for use in the treatment of a disease of the carbohydrate metabolism, more preferred of diabetes, more preferred of diabetes mellitus type 2, and most preferred for the up-regulation of insulin production.

The invention refers further to a method for screening for a modulator for use in the treatment of a metabolic disease, wherein the method comprises providing a test compound for contacting at least one polypeptide or nucleic acid coding for the GRK5 polypeptide, detecting the binding of said test compound to the GRK5 polypeptide and determining the activity of the GRK5 polypeptide in the presence of said test compound, wherein said last step comprises determining a change of the insulin release level compared to the level without the test compound.

For identification of an inventive compound the invention further provides a kit for a method to screen for a modulator for use in the treatment of a disease of the carbohydrate metabolism comprising the GRK5 polypeptides, the nucleic acid encoding GRK5, a cell line with a glucose dependent insulin production, and a control compound known to affect the insulin production by binding the GRK5 polypeptide.

### Description

It was surprisingly found that Sunitinib has an effect on insulin release in a dose dependent manner. It was found that this effect was due to the inhibition of certain kinases by Sunitinib. It was further found that the combined inhibition of certain protein kinase pairs results in the release of additional insulin. It was further found that the inhibition of certain protein kinase pairs results in an insulin releasing effect which equals the effect after Sunitinib treatment.

Kinases are enzymes which catalyze the transfer of a phosphate group from a donor onto an acceptor. Phosphorylated is a nucleophil functional group, such as hydroxyl-, carboxy-, guanidino-, thiol-, or imidazole groups. Kinases which phosphorylate proteins are called protein kinases. Protein kinases play a particular role in cellular signal transduction. They are usually categorized by their substrate; thus protein kinases may be roughly divided into two groups: protein tyrosine kinases (PTK), which phosphorylate the hydroxyl group of the tyrosine, and serine/threonine kinases (STK), which phosphorylate the hydroxyl groups of the serine or threonine. Examples for PTK are Kinases of the EphA family, Lck, Scyl, HCK, BLK, ITK, TEC, EXK, BTK, CTK, Fyn, Fgr, Src, Yes, Lyn, Tyk, JAK-family, CSK, Arg, Abl, Fes, Fer, Srm, Brk, Syk, ZAP70, FAK, PYK2, DDR, TRK, HER-family, FGFR-family, FLT, Mer, Reg, Axl, Met, Ron, RYK, InsR, IGF1R, LTK, ALK, Ros, Lmr-family. STK are mainly regulated by cAMP, cGMP, DAG, Ca²⁺ or Calmodulin, 1,2-Diacylglycerine, PIP3 and other phospholipid-derivates. Examples for STK include enzymes of the families protein kinase A, B and C, GRK-family, MAST-family, CSNK, PRK, NDR, p70S6K, MSK, MRCK, ROCK, CRIK, DMPK, PKN, Nek, Pim, Aur, SSTK, TSSK, Obscn, skMLCK, DRAK, FAPK, BRSK, MNK, PKD, MAP, PIK3, CHBK, PIP, CERK, TLK, CASK, AKT, KCNH2, GSK, FUK.

Other categorizations are based on the activating compounds of the kinases, or the activating mechanism, or certain catalytic domains or specific amino acid sequences of the kinases. The sum of all kinases in one cell is called kinome.

Based on their function the protein kinases present a very important control mechanism in signal transduction, and are controlling various anabolic and metabolic pathways. On the basis of their importance dysfunctions of protein kinases in cellular pathways are the cause for many diseases, like cancer, metabolic diseases, cardiovascular diseases, arteriosclerosis, thyroid disorders, endocrinological diseases, gastroenterological diseases, inflammation, immune disorders, disorders affecting growth and development, hematological diseases, respiratory diseases, muscle skeleton diseases, neurological diseases, and urological disease. This makes these enzymes attractive molecular targets for therapy.

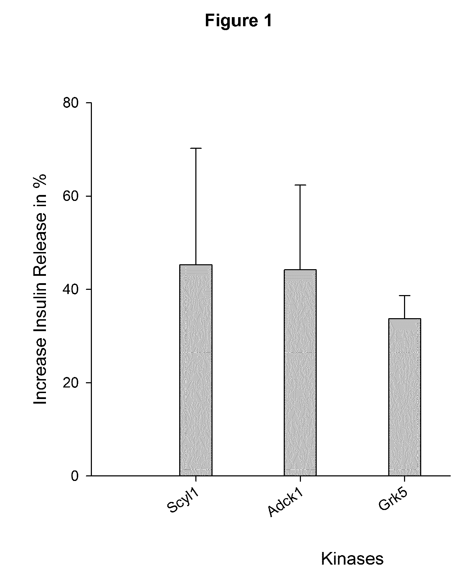

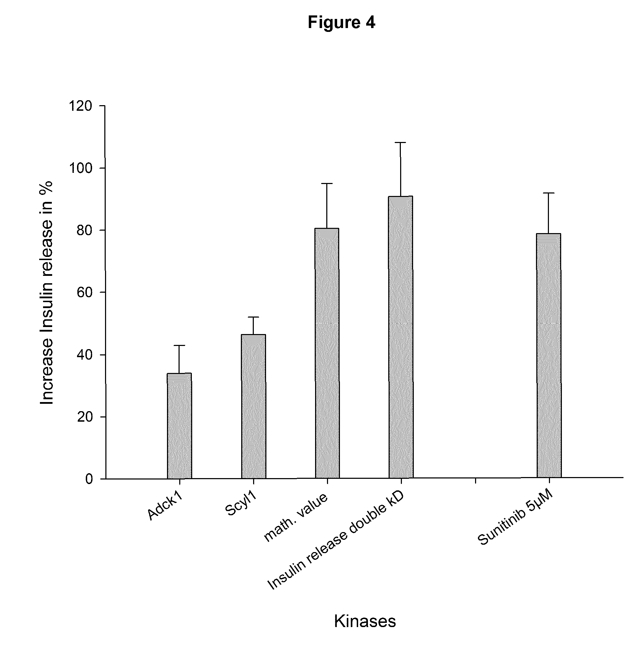

Surprisingly the kinases SCYL1, ADCK1, and GRK5 were identified to modulate the insulin release in a significant manner (Fig. 1 and 4). These kinases are preferred targets for the therapy of metabolic disease, preferred of diabetes, more preferred of diabetes type 2, and most preferred to elevate the blood level of insulin. Kinases which present a target for the therapy of metabolic diseases are basically all kinases which affect metabolic pathways. Kinases which have been found to affect or modulate the insulin release or sensitivity and are thus preferred targets for a therapy of diabetes, preferably diabetes type 2, are kinases the inhibition of which is correlated to a significant increase of insulin release, namely SCYL1, ADCK1, and GRK5.

The present invention refers particularly to a modulator for
a) inhibition of the protein kinase GRK5, or
b) inactivation, degradation, downregulation, intercalation of the nucleic acid encoding GRK5,
   for use in the treatment of a disease of the carbohydrate metabolism,
   wherein the modulator is a small molecule, an siRNA molecule, or an antisense oligonucleotide and wherein the modulator is not sunitinib.

Thus, in a preferred embodiment of the invention, the action of the protein kinase GRK5 is blocked by a modulator or even more preferred by an inhibitor.

Hence, the present invention refers preferred to an inhibitor for
a) the protein kinase GRK5, or
b) inactivation, degradation, downregulation or intercalation of the nucleic acid encoding GRK5,
for use in the treatment of disease of the carbohydrate metabolism, wherein the modulator is a small molecule, an siRNA molecule, or an antisense oligonucleotide and wherein the modulator is not sunitinib.

It is sufficient to block one of the kinases SCYL1, ADCK1, or GRK5. However, inhibition of two kinases of SCYL1, ADCK1, GRK5 may further increase the therapeutic effect. In a further preferred embodiment of the invention, all three enzymes SCYL1, ADCK1, and GRK5 may be inhibited at once for an increased insulin release. In order to achieve the inhibition of two kinases a single modulator or inhibitor or a combination of two modulators or inhibitors could be used. In order to achieve the inhibition of the three kinases SCYL1, ADCK1, and GRK5 a single modulator or inhibitor or a combination of two modulators or inhibitors or a combination of three inhibitors could be used. Enzyme inhibitors are, in general, molecules which bind to enzymes and decrease their activity. The binding of an inhibitor can stop a substrate from entering the enzymes active site, compete with the substrate for the binding site, or hinder the enzyme from catalyzing its reaction. Inhibitor binding can be reversible or irreversible. Protein kinase inhibitors are a type of enzyme inhibitors which specifically block the action of one or more protein kinases. Inhibition of protein kinases can be achieved using a pseudosubstrate binding to the active site of these kinases mimicking the target sequence of the corresponding kinase, but having no serine or threonine.

In another preferred embodiment, the action of the protein kinase GRK5 is impeded by interference of their nucleic acid, which can be both DNA and RNA, by inactivation, degradation, downregulation, or intercalation. Inactivation of a nucleic acid can happen for instance by methylation of nucleotides, insertion, deletion, nucleotide exchange, cross linkage, or strand break/damage. Nucleic acids can be degraded down to single nucleotides by temperature, chemicals, enzymes, and particularly RNA by deadenylation or 5'decay or 3'decay. Downregulation of DNA or RNA is referred to as diminished expression of these nucleic acids and can happen by binding of repressors, which are usually polypeptides, but can also happen by chemical or structural changes or modifications of the nucleic acids. Intercalation is the reversible inclusion of a molecule between two other molecules. In nucleic acids, intercalation occurs when ligands of an appropriate size and chemical nature fit themselves in between base pairs.

The term modulator as it appears herein refers to a molecule that is able to change the activity of the SCYL1, ADCK1, and/or GRK5 polypeptides. This change may in general be an increase or a decrease in enzymatic activity, binding characteristics, or functional, immunological or any other biological property of the polypeptides. In order to enhance the insulin release, a decrease of the enzymatic activity is advantageous.

Modulators for the inhibition of SCYL1, ADCK1, and GRK5 can be molecules like small molecules, siRNA or precursor thereof, DNA or RNA antisense oligonucleotides, or drugs like imatinib, dasanitib, and sorafenib.

The inventive modulators are also referred to as compounds or test compounds. They modulate the expression and/or activity of the GRK5 polypeptide and can be identified using one or more assays, alone or in combination. Test compounds used in the screening are not particularly limited. They can be either artificial or natural.

The term small molecule refers to low molecular weight organic compound which is by definition not a polymer. In the field of pharmacology, it is usually restricted to a molecule that also binds with high affinity to a biopolymer such as proteins, nucleic acids, or polysaccharides. The upper molecular weight limit for a small molecule is approximately 200 Da which allows for the possibility to rapidly diffuse across cell membranes. Small molecules are broadly used as enzyme inhibitors, thus they are preferred modulators for the inhibition of the kinases in the present invention.

Small interfering RNA (short interfering RNA, silencing RNA, siRNA) is a class of double-stranded RNA-molecules, which are 19-30 nucleotides, preferably 20-25 nucleotides long. SiRNAs are involved in the RNA-interference of the expression of a specific gene. SiRNAs are cut from long doublestranded RNAs by the RNase III Dicer. They can also be derived by chemical synthesis. They also play a role in antiviral mechanisms or in shaping the chromatin structure of a genome. In molecular research, synthetic siRNAs can also be used in RNA-interference (RNAi) to regulate down the expression of specific target genes. With their ability to knock down essentially any gene of interest, siRNAs have been used to knock down protein kinases to investigate their role in insulin production (Fig. 1 - Fig. 4). SiRNAs are preferred modulators for inhibition of the kinases in the present invention.

Precursor molecules, e.g. precursor molecules of siRNA may be a substrate for the siRNA -biogenesis-apparatus of the target cell. This comprises, for example, RNA precursor molecules such as double-stranded RNA (dsRNA) or short hairpin RNA-molecules (shRNA), which are processed by endonucleases such as Drosha and/or Pasha to siRNA-molecules. For this reason, for example dsRNA-molecules or short hairpin RNA-molecules (shRNA) having a length of more than 27 nucleotides, preferably more than 30 up to 100 nucleotides or longer, and mostly preferred dsRNA-molecules having a length of 30-50 nucleotides, can be used.

Further precursor molecules according to the invention may be DNA constructs encoding dsRNA, shRNA, and/or siRNA, whereby the coding elements are controlled by regulatory elements allowing an expression of dsRNA, shRNA, or siRNA in the target cell. Examples for such control elements are polymerase II promoters or polymerase III promoters such as, for example, U6 or H1.

Antisense oligonucleotides are single strands of DNA or RNA that are complementary to a chosen sequence. They are between 10 and 35 nucleotides long, preferably about 20 - 25 nucleotides. Antisense DNA oligonucleotides can target specific, complementary RNA, and upon binding DNA/RNA hybrids are formed. Antisense RNA oligonucleotides can bind to mRNA by binding to mRNA strands. Antisense oligonucleotides are also preferred modulators for inhibition of the kinases in the present invention.

Peptides are stretches of amino acid residues which are connected by peptide bonds. They can be seen as little proteins. Peptides are usually up to 100 amino acids long, from which on the compound is referred to as a protein. Polypeptides are peptides of at least 10 amino acids. Cyclopeptides are formed by two, three or more amino acids, which form ring structures and have thus no C- and N-terminal amino acids. Peptides are preferred, polypeptides more preferred modulators for inhibition of the kinase in the present invention.

The drugs sunitinib, imatinib, dasatinib, and sorafenib are small molecules which inhibit protein kinases which are mainly used in cancer treatment. However, in the present invention the drug sunitinib is not a claimed modulator for inhibition of the kinase as one major side effect under sunitinib treatment is high blood pressure. People who have diabetes tend to have more trouble with high blood pressure than people who don't have the disease. Having both diabetes and high blood pressure can pack a damaging one-two punch as far as increasing the risk of heart disease, stroke, and eye, kidney and nerve complications. There are particularly common diabetes complications associated with elevated blood pressure. These complications include diabetic retinopathy and diabetic nephropathy. Controlling blood pressure of people with diabetes reduces the risk of future complications as established by a study done by the UK Prospective Diabetes Study.

Metabolic diseases refer to diseases and conditions characterized by pathological disorders of the metabolism. They are mainly characterized by enzyme defects and abnormalities in the regulating system leading to a pathological enrichment of substrates, lack of metabolic products, failure of producing energy, of regeneration of cellular constituents, of elimination of metabolic products, and of maintenance of homeostasis. They can be acquired or be a genetic disease. Metabolic disorders include, but are not limited to, obesity and diabetes (e.g., diabetes type I, diabetes type II, MODY, and gestational diabetes), hypoglycemia, amyloidosis, branched chain disease, hyperaminoacidemia, hyperaminoaciduria, disturbances of the metabolism of urea, hyperammonemia, mucopolysaccharidoses e. g. Maroteaux-Lamy syndrom, glycogen storage diseases and lipid storage diseases, Cori's disease, intestinal carbohydrate malabsorption, maltase-, lactase-, sucrase-insufficiency, disorders of the metabolism of fructose, disorders of the metabolism of galactose, galactosaemia, disturbances of pyruvate metabolism, hypolipidemia, hypolipoproteinemia, hyperlipidemia, hyperlipoproteinemia, camitine or camitine acyltransferase deficiency, porphyrias, disturbances of the purine metabolism, lysosomal diseases, metabolic diseases of nerves and nervous systems like gangliosidoses, sphingolipidoses, sulfatidoses, leucodystrophies, Lesch-Nyhan syndrome, dysfunction of the parathyroid glands, pancreatic islet cell dysfunction, carbohydrate and lipid storage myopathies, glycogenoses, myoglobinuria, alkaptonuria, adrenogenital syndrome, ketosis, ketoacidosis, methylmalonaciduria, Morbus Addison, Morbus Conn, Morbus Cushing, Morbus Fabry, Morbus Gaucher, Morbus Hunter, cystic fibrosis, phenylketonuria, thesaurismosis, uricopathia. Carbohydrate metabolism denotes the various biochemical processes responsible for the formation, breakdown and interconversion of carbohydrates in living organisms, wherein the most important carbohydrate is glucose. The hormone insulin is the primary regulatory signal in animals; when present, it causes many tissue cells to take up glucose from the circulation, causes some cells to store glucose internally in the form of glycogen, causes some cells to take in and hold lipids, and in many cases controls cellular electrolyte balances and amino acid uptake as well. Diseases of the carbohydrate metabolism refer to diseases and conditions characterized in pathophysiological alterations in the metabolism of one or more carbohydrates. It is preferred if the disease of the carbohydrate metabolism is chosen of one disease of the group comprising or consisting of Diabetes mellitus, Lactose intolerance, Fructose intolerance, Galactosemia, Glycogen storage disease, diabetic ketoacidosis, hyperosmolar coma and hypoglycemia.

The invention relates also to pharmaceutical compositions comprising or consisting of an effective amount of at least one inventive compound, and at least one pharmaceutically acceptable carrier, excipient, binders, disintegrates, glidents, diluents, lubricants, coloring agents, sweetening agents, flavoring agents, preservatives, solvent or the like for use in the treatment of a disease of the carbohydrate metabolism. The pharmaceutical compositions of the present invention can be prepared in a conventional solid or liquid carrier or diluents and a conventional pharmaceutically-made adjuvant at suitable dosage level in a known way.

According to the invention, the inventive compound or the pharmaceutical composition can be used for the treatment of diseases of the carbohydrate metabolism, preferably of diabetes mellitus, more preferably of diabetes mellitus type 2, and most preferably to increase the level of insulin release from pancreas cells.

The inventive pharmaceutical composition is formulated to be compatible with its intended route of administration. Administration forms include, for example, pills, tablets, film tablets, coated tablets, capsules, liposomal formulations, micro- and nano-formulations, powders and deposits. Furthermore, the present invention also includes pharmaceutical preparations for parenteral application, including dermal, intradermal, intragastral, intracutan, intravasal, intravenous, intramuscular, intraperitoneal, intranasal, intravaginal, intrabuccal, percutan, rectal, subcutaneous, sublingual, topical, or transdermal application, which preparations in addition to typical vehicles and/or diluents contain the compound according to the present invention. Intravenous and oral applications are preferred forms of administration in the present invention, wherein oral application is particularly preferred.

The present invention also includes the mammalian milk, artificial mammalian milk as well as mammalian milk substitutes as a formulation for oral administration of the inventive compound to newborns, toddlers, and infants, either as pharmaceutical preparations, and/or as dietary food supplements.

The inventive compound can also be administered in form of its pharmaceutically active salts. Suitable pharmaceutically active salts comprise acid addition salts and alkali or earth alkali salts. For instance, sodium, potassium, lithium, magnesium or calcium salts can be obtained.

The pharmaceutical compositions according to the present invention will typically be administered together with suitable carrier materials selected with respect to the intended form of administration, i.e. for oral administration in the form of tablets, capsules (either solid filled, semi-solid filled or liquid filled), powders for constitution, aerosol preparations consistent with conventional pharmaceutical practices. Other suitable formulations are gels, elixirs, dispersible granules, syrups, suspensions, creams, lotions, solutions, emulsions, suspensions, dispersions, and the like. Suitable dosage forms for sustained release include tablets having layers of varying disintegration rates or controlled release polymeric matrices impregnated with the active components and shaped in tablet form or capsules containing such impregnated or encapsulated porous polymeric matrices. The pharmaceutical compositions may be comprised of 5 to 95% by weight of the inventive compound.

As pharmaceutically acceptable carrier, excipient and/or diluents can be used lactose, starch, sucrose, cellulose, magnesium stearate, dicalcium phosphate, calcium sulfate, talc, mannitol, ethyl alcohol (liquid filled capsules).

Suitable binders include starch, gelatin, natural sugars, corn sweeteners, natural and synthetic gums such as acacia, sodium alginate, carboxymethyl-cellulose, polyethylene glycol and waxes. Among the lubricants that may be mentioned for use in these dosage forms, boric acid, sodium benzoate, sodium acetate, sodium chloride, and the like. Disintegrants include starch, methylcellulose, guar gum and the like. Sweetening and flavoring agents and preservatives may also be included where appropriate. Some of the terms noted above, namely disintegrants, diluents, lubricants, binders and the like, are discussed in more detail below.

Additionally, the compositions or modulators of the present invention may be formulated in sustained release form to provide the rate controlled release of any one or more of the components or active ingredients to optimize the therapeutic effects. Suitable dosage forms for sustained release include layered tablets containing layers of varying disintegration rates or controlled release polymeric matrices impregnated with the active components and shaped in tablet form or capsules containing such impregnated or encapsulated porous polymeric matrices.

Aerosol preparations suitable for inhalation may include solutions and solids in powder form, which may be in combination with a pharmaceutically acceptable carrier such as inert compressed gas, e.g. nitrogen.

For preparing suppositories, a low melting wax such as a mixture of fatty acid glycerides such as cocoa butter is first melted, and the active ingredient is dispersed homogeneously therein by stirring or similar mixing. The molten homogeneous mixture is then poured into convenient sized molds, allowed to cool and thereby solidify.

Also included are solid form preparations which are intended to be converted, shortly before use, to liquid form preparations for either oral or parenteral administration. Such liquid forms include solutions, suspensions and emulsions.

The inventive compound may also be deliverable transdermally. The transdermal compositions may take the form of creams, lotions, aerosols and/or emulsions and can be included in a transdermal patch of the matrix or reservoir type as are conventional in the art for this purpose.

The term capsule refers to a special container or enclosure made of methyl cellulose, polyvinyl alcohols, or denatured gelatins or starch for holding or containing compositions comprising the active ingredients. Hard shell capsules are typically made of blends of relatively high gel strength bone and pork skin gelatins. The capsule itself may contain small amounts of dyes, opaquing agents, plasticizers and preservatives.

Tablet means compressed or molded solid dosage form containing the active ingredients with suitable diluents. The tablet can be prepared by compression of mixtures or granulations obtained by wet granulation, dry granulation or by compaction well known to a person skilled in the art.

Oral gels refer to the active ingredients dispersed or solubilized in a hydrophilic semi-solid matrix.

Powders for constitution refer to powder blends containing the active ingredients and suitable diluents which can be suspended in water or juices. One example for such an oral administration form for newborns, toddlers and/or infants is a human breast milk substitute which is produced from milk powder and milk whey powder, optionally and partially substituted with lactose.

Human breast milk is a complex fluid, rich in nutrients and in non-nutritional bioactive components. It contains all of the nutrients needed by the newborn baby. These include the metabolic components (fat, protein, and carbohydrates), water, and the raw materials for tissue growth and development, such as fatty acids, amino acids, minerals, vitamins, and trace elements.

More than 98% of the fat is in the form of triglycerides. Oleic acid and palmitic acid are the most abundant fatty acids in breastmilk triglycerides, with comparatively high proportions of the essential fatty acids, and linolenic acid, followed by long-chain polyunsaturated fatty acids, such as arachidonic acid and docosahexaenoic acid. These long-chain fatty acids are constituents of brain and neural tissue and are needed in early life for mental and visual development. The lipid component of breast milk is the transport vehicle for fat-soluble micronutrients such as prostaglandins and vitamins A, D, E, and K.

Proteins account for approximately 75 % of the nitrogen-containing compounds in breast milk. Non-protein nitrogen substances include urea, nucleotides, peptides, free amino acids, and DNA. The proteins of breast milk can be divided into two categories: micellar caseins and aqueous whey proteins, present in the ratio of about 40:60. Casein forms micelles of relatively small volume and produces a soft, flocculent curd in the infant's stomach. The major whey proteins are lactalbumin, lactoferrin, secretory IgA, and serum albumin, with a large number of other proteins and peptides present in smaller amounts.

The principal carbohydrate is lactose, a disaccharide produced in the mammary epithelial cell from glucose by a reaction involving lactalbumin.

In addition to the nutritional components, breast milk contains a wealth of bioactive components that have beneficial non-nutritional functions. These include a wide range of specific and non-specific antimicrobial factors; cytokines and anti-inflammatory substances; and hormones, growth modulators, and digestive enzymes, many of which have multiple activities. These components may be of particular importance for young infants because of the immaturity of the host defense and digestive systems early in life.

The artificial mother milk formulations or mother milk substitutes of the present invention are preferably prepared by adding to a mother milk formulation including commercially available mother milk formulations especially in powder form of the compound of the present invention. The inventive compound is preferably added in an amount of 3 - 100 µg compound or per 100 ml (commercially available) mother milk formulation, more preferably in an amount of 5 - 70 µg / 100 ml and most preferably in an amount of 10 - 40 µg / 100 ml mother milk formulation.

Suitable diluents are substances that usually make up the major portion of the composition or dosage form. Suitable diluents include sugars such as lactose, sucrose, mannitol and sorbitol, starches derived from wheat, corn rice and potato, and celluloses such as microcrystalline cellulose. The amount of diluents in the composition can range from about 5 to about 95% by weight of the total composition, preferably from about 25 to about 75%, more preferably from about 30 to about 60% by weight, and most preferably from about 40 to 50% by weight.

The term disintegrants refers to materials added to the composition to help it break apart (disintegrate) and release the medicaments. Suitable disintegrants include starches, "cold water soluble" modified starches such as sodium carboxymethyl starch, natural and synthetic gums such as locust bean, karaya, guar, tragacanth and agar, cellulose derivatives such as methylcellulose and sodium carboxymethylcellulose, microcrystalline celluloses and cross-linked microcrystalline celluloses such as sodium croscarmellose, alginates such as alginic acid and sodium alginate, clays such as bentonites, and effervescent mixtures. The amount of disintegrant in the composition can range from about 1 to about 40% by weight of the composition, preferably 2 to about 30% by weight of the composition, more preferably from about 3 to 20% by weight of the composition, and most preferably from about 5 to about 10% by weight.

Binders characterize substances that bind or "glue" powders together and make them cohesive by forming granules, thus serving as the "adhesive" in the formulation. Binders add cohesive strength already available in the diluents or bulking agent. Suitable binders include sugars such as sucrose, starches derived from wheat, corn rice and potato; natural gums such as acacia, gelatin and tragacanth; derivatives of seaweed such as alginic acid, sodium alginate and ammonium calcium alginate; cellulosic materials such as methylcellulose and sodium carboxymethylcellulose and hydroxypropyl-methylcellulose; polyvinylpyrrolidone; and inorganics such as magnesium aluminum silicate. The amount of binder in the composition can range from about 1 to 30% by weight of the composition, preferably from about 2 to about 20% by weight of the composition, more preferably from about 3 to about 10% by weight, even more preferably from about 3 to about 6% by weight.

Lubricant refers to a substance added to the dosage form to enable the tablet, granules, etc. after it has been compressed, to release from the mold or die by reducing friction or wear. Suitable lubricants include metallic stearates such as magnesium stearate, calcium stearate or potassium stearate; stearic acid; high melting point waxes; and water soluble lubricants such as sodium chloride, sodium benzoate, sodium acetate, sodium oleate, polyethylene glycols and d'I-leucine. Lubricants are usually added at the very last step before compression, since they must be present on the surfaces of the granules and in between them and the parts of the tablet press. The amount of lubricant in the composition can range from about 0.05 to about 15% by weight of the composition, preferably 0.2 to about 5% by weight of the composition, more preferably from about 0.3 to about 3%, and most preferably from about 0.3 to about 1.5% by weight of the composition.

Glidents are materials that prevent caking and improve the flow characteristics of granulations, so that flow is smooth and uniform. Suitable glidents include silicon dioxide and talc. The amount of glident in the composition can range from about 0.01 to 10% by weight of the composition, preferably 0.1 % to about 7% by weight of the total composition, more preferably from about 0.2 to 5% by weight, and most preferably from about 0.5 to about 2% by weight.

Coloring agents are excipients that provide coloration to the composition or the dosage form. Such excipients can include food grade dyes and food grade dyes adsorbed onto a suitable adsorbent such as clay or aluminum oxide. The amount of the coloring agent can vary from about 0.01 to 10% by weight of the composition, preferably from about 0.05 to 6% by weight, more preferably from about 0.1 to about 4% by weight of the composition, and most preferably from about 0.1 to about 1%.

Liquid form preparations include solutions, suspensions and emulsions. As an example may be mentioned water or water-propylene glycol solutions for parenteral injections or addition of sweeteners and opacifiers for oral solutions, suspensions and emulsions. Liquid form preparations may also include solutions for intranasal administration.

Other preferred pharmaceutical compositions are buffered solutions. The term buffer, buffer system, buffer solution and buffered solution, when used with reference to hydrogen-ion concentration or pH, refers to the ability of a system, particularly an aqueous solution, to resist a change of pH on adding acid or alkali, or on dilution with a solvent. Preferred buffer systems can be selected from the group consisting of formate (pKa=3.75), lactate (pKa=3.86), benzoic acid (pKa=4.2) oxalate (pKa=4.29), fumarate (pKa=4.38), aniline (pKa=4.63), acetate buffer (pKa=4.76), citrate buffer (pKa2=4.76,pKa3=6.4), glutamate buffer (pKa=4.3), phosphate buffer (pKa=7.20), succinate (pKa1=4.93;pKa2=5.62), pyridine (pKa=5.23), phthalate (pKa=5.41); histidine (pKa=6.04), MES (2-(N-morpholino)ethanesulphonic acid; pKa=6.15); maleic acid (pKa=6.26); cacodylate (dimethylarsinate, pKa=6.27), carbonic acid (pKa=6.35), ADA (N-(2-acetamido)imino-diacetic acid (pKa=6.62); PIPES (4-piperazinebis-(ethanesulfonic acid; BIS-TRIS-propane (1,3-bis[tris(hydroxymethyl)methylamino]-propane), pKa=6.80), ethylendiamine (pKa=6.85), ACES 2-[(2-amino-2-oxoethyl)amino]ethanesulphonic acid; pKa=6.9), imidazole (pKa=6.95), MOPS (3-(N-morphin)-propansulfonic acid; pKa=7.20), diethylmalonic acid (pKa=7.2), TES (2-[tris (hydroxymethyl) methyl] amino ethanesulphonic acid; pKa=7.50) and HEPES (N-2-hydroxylethylpiperazin-N'-2-ethansulfonic acid; pKa=7.55) buffers or other buffers having a pKa between 3.8 to 7.7.

Preferred is the group of carboxylic acid buffers such as acetate and carboxylic diacid buffers such as fumarate, tartrate and phthalate and carboxylic triacid buffers such as citrate. Another group of preferred buffers is represented by inorganic buffers such as sulfate, borate, carbonate, oxalate, calcium hydroxyde and phosphate buffers. Another group of preferred buffers are nitrogen containing buffers such as imidazole, diethylenediamine, and piperazine.

Also preferred are sulfonic acid buffers such as TES, HEPES, ACES, PIPES, [(2-hydroxy-1,1-bis(hydroxymethyl)ethyl)amino]-1-propanesulfonic acid (TAPS), 4-(2-hydroxyethyl)piperazine-1-propanesulfonic acid (EPPS), 4-Morpholinepropanesulfonic acid (MOPS) and N,N-bis(2-hydroxyethyl)-2-aminoethanesulfonic acid (BES).

Another group of preferred buffers are glycine buffers such as glycine, glycyl-glycine, glycyl-glycyl-glycine, N,N-bis(2-hydroxyethyl)glycine and N-[2-hydroxy-1,1-bis(hydroxy-methyl)ethyl]glycine (Tricine).

Preferred are also amino acid buffers such as glycine, alanine, valine, leucine, isoleucine, serine, threonine, phenylalanine, tyrosine, tryptophane, lysine, arginine, histidine, aspartate, glutamate, asparagine, glutamine, cysteine, methionine, proline, 4-hydroxyproline, N,N,N-trimethyllysine, 3-methylhistidine, 5-hydroxylysine, O-phosphoserine, γ-carboxyglutamate, ε-N-acetyllysine, ω-N-methylarginine, citrulline, ornithine and derivatives thereof.

Preferred are the buffers having an effective pH range of from 2.7 to 8.5, and more preferred of from 3.8 to 7.7. The effective pH range for each buffer can be defined as pKa - 1 to pKa + 1, where Ka is the ionization constant for the weak acid in the buffer and pKa = - log K.

Most preferred are buffers suitable for pharmaceutical use e.g. buffers suitable for administration to a patient such as acetate, carbonate, citrate, fumarate, glutamate, lactate, phosphate, phthalate, and succinate buffers. Particularly preferred examples of commonly used pharmaceutical buffers are acetate buffer, citrate buffer, glutamate buffer and phosphate buffer. Also most preferred is the group of carboxylic acid buffers. The term "carboxylic acid buffers" as used herein shall refer to carboxylic mono acid buffers and carboxylic diacid buffers as well as carboxylic triacid buffers. Of course also combinations of buffers, especially of the buffers mentioned herein are useful for the present invention.

Some suitable pharmaceutical buffers are a citrate buffer (preferably at a final formulation concentration of from about 20 to 200 mM, more preferably at a final concentration of from about 30 to 120 mM) or an acetate buffer (preferably at a final formulation concentration of about 20 to 200 mM) or a phosphate buffer (preferably at a final formulation concentration of about 20 to 200 mM).

Techniques for the formulation and administration of the compound of the present invention may be found in "Remington's Pharmaceutical Sciences" Mack Publishing Co., Easton PA. A suitable composition comprising the compound mentioned herein may be a solution of the compound in a suitable liquid pharmaceutical carrier or any other formulation such as tablets, pills, film tablets, coated tablets, dragees, capsules, powders and deposits, gels, syrups, slurries, suspensions, emulsions, and the like.

A particularly preferred pharmaceutical composition is a lyophilised (freeze-dried) preparation (lyophilisate) suitable for administration by inhalation or for intravenous administration. To prepare the preferred lyophilised preparation the compound of the invention is solubilised in a 4 to 5% (w/v) mannitol solution and the solution is then lyophilised. The mannitol solution can also be prepared in a suitable buffer solution as described above.

Further examples of suitable cryo- / lyoprotectants (otherwise referred to as bulking agents or stabilizers) include thiol-free albumin, immunoglobulins, polyalkyleneoxides (e.g. PEG, polypropylene glycols), trehalose, glucose, sucrose, sorbitol, dextran, maltose, raffinose, stachyose and other saccharides (cf. for instance WO 97/29782), while mannitol is used preferably. These can be used in conventional amounts in conventional lyophilization techniques. Methods of lyophilisation are well known in the art of preparing pharmaceutical formulations.

For administration by inhalation the particle diameter of the lyophilised preparation is preferably between 2 to 5 µm, more preferably between 3 to 4 µm. The lyophilised preparation is particularly suitable for administration using an inhalator, for example the OPTINEB^{®} or VENTA-NEB^{®} inhalator (NEBU-TEC, Elsenfeld, Germany). The lyophilised product can be rehydrated in sterile distilled water or any other suitable liquid for inhalation adminstration.

Alternatively for intravenous administration the lyophilised product can be rehydrated in sterile distilled water or any other suitable liquid for intravenous administration.

After rehydration for administration in sterile distilled water or another suitable liquid the lyophilised preparation should have the approximate physiological osmolality of the target tissue for the rehydrated compound preparation i.e. blood for intravenous administration or lung tissue for inhalation administration. Thus it is preferred that the rehydrated formulation is substantially isotonic.

The preferred dosage concentration for either intravenous, oral, or inhalation administration is between 100 to 2000 µmol/ml, and more preferably is between 200 to 800 µmol/ml. These are also the preferred ranges of the compound in the mother milk substitute or artificial mother milk formulation or the pharmaceutical compositions disclosed herein.

Still another aspect of the present invention relates to the use of the inventive compound as a dietary supplement. That dietary supplement is preferably for oral administration and especially but not limited to administration to newborns, toddlers, and/or infants. A dietary supplement is intended to supplement the diet. The "dietary ingredients" in these products may in addition include: vitamins, minerals, herbs or other botanicals, amino acids, and substances such as enzymes, organ tissues, glandulars, and metabolites. Dietary supplements may be manufactured in forms such as tablets, capsules, softgels, gelcaps, liquids, or powders.

The invention further relates to a method for screening for a modulator for treatment of a metabolic disease, the method comprising
a) contacting a test compound with a GRK5 polypeptide,
b) detecting the binding of said test compound to the GRK5 polypeptide, and
c) determining the activity of the GRK5 polypeptide in the presence of said test compound, wherein step c) comprises determining a change of the insulin release level compared to the level without the test compound.

The screening method of the present invention apparently consists of three steps. The term test compound may be any of the potential modulators listed above. The contacting of the test compound with at least one of the polypeptides can happen e.g. in the form of a compound library, in physiological or non-physiological solution, or solid phase systems, however a liquid environment is preferred. The conditions and the time need to be sufficient to allow the test compound to bind to the polypeptide(s). The method is normally carried our in solution at room temperature and at a suitable pH value normally between pH 5 and 9, all parameters which are easily selected by a skilled person.

The polypeptide GRK5 can be obtained by purification from primary human cells, cell lines, from cells which have been transfected with expression constructs which contain the nucleic acid sequence encoding the polypeptide GRK5 , or by direct chemical synthesis.

The nucleic acid sequences encoding the polypeptide GRK5 can be obtained by cloning the relevant genes, amplification of the cDNAs or chemical synthesis of the nucleic sequences. For the expression of the corresponding polypeptides the nucleic acid sequences can be inserted into expression vectors, such as recombinant bacteriophage, plasmid, or cosmid DNA expression vectors.

The term binding refers to an interaction between the test compound and the polypeptide GRK5 or the nucleic acid encoding the polypeptide GRK5. For binding to a protein, the binding interaction is dependent upon the presence of a particular structure of the kinase, e.g. the antigenic determinant or epitope, recognized by the binding molecule. For binding of compounds to nucleic acids, test compounds need to have a complementary sequence to the nucleic acids, or fit into certain secondary or tertiary structures of the nucleic acids.

The binding of the test compounds to the polypeptide or nucleic acid can be checked by any convenient method known in the art. A separation step may be included to separate bound from unbound components. To check whether the test compound has been bound by the polypeptide or nucleic acid, it is advantageous if the test compound is labeled for direct detection (radioactivity, luminescence, fluorescence, optical or electron density etc.) or indirect detection (e.g., epitope tag such as the FLAG, V5 or *myc* epitopes, an enzyme tag such as horseradish peroxidase or luciferase, a transcription product, etc.). The label may be bound to a substrate, to the proteins employed in the assays, or to the candidate pharmacological agent. The binding of a test compound can also be conveniently checked if one of the components is immobilized on a solid substrate. The substrate can be made of a wide variety of materials and in various shapes, e.g. tubes, microtiter plates, microbeads, dipsticks and the like. It is also advantageous if one of the components is modified by biotinylation, so that the components can be immobilized on streptavidin-covered surfaces.

Protein-DNA interactions can be for instance checked by gel shift or band shift assays or elektrophoretic mobility shift assays (EMSA), which is based on the observation that complexes of protein and DNA migrate through a non-denaturing polyacrylamide gel more slowly that a free DNA fragments.

Protein-RNA interactions can be investigated by RNA electrophoretic mobility shift assays which are an *in vitro* technique used to detect protein-RNA interactions through changes in migration speed during gel electrophoresis. After incubation, the binding reaction is then separated via non-denaturing polyacrylamide gel electrophoresis. Like protein-DNA complexes, a protein-RNA complex migrates more slowly than a free RNA probe through a gel matrix. This causes a migration shift relative to the nonbound RNA probe. Specificity is determined through a competition reaction, where excess unlabeled RNA is incubated in the binding reaction, resulting in a decrease in the shifted signal if the labeled and unlabeled RNA sequences compete for binding of the same protein. Alternatively, the protein-RNA complex may be crosslinked and the reaction run on a denaturing gel. Specificity is determined through visualization of a single shifted band. Traditionally, RNA probes are radioactively labeled for detection, although fluorescent and chemiluminescent detection is also possible. Non-radioactive RNA end-labeling techniques are limited, but more versatile biotin and fluorescent labeling methods are now available. Alternatively, RNA Pull-down assays can be carried out which selectively extract a Protein-RNA complex from a sample. This method has the advantage that several RNAs can be used with the target protein(s), and selectively binding RNAs can be identified. Typically, the RNA pull-down assay takes advantage of high affinity tags, such as biotin or azido-phosphine chemistry. RNA probes can be biotinylated, complexed with a protein from a cell lysate and then purified using agarose or magnetic beads. Alternatively, the protein may be labeled, or the RNA-Protein complex may be isolated using an antibody against the protein of interest. The RNA is then detected by Northern blot or through RT-PCR analysis and the proteins detected by Western blotting or mass spectrometry. Protein-RNA interactions can also be identified by oligonucleotide-targeted RNase H protection assays (RPA), which is a powerful method for detecting RNA and RNA fragments in cell extracts. Unlike Northern blotting or RT-PCR analysis, RPA assays allow greater flexibility in the integrity of target RNA, requiring very short segments for hybridization and detection. RPA assays can also be used to map protein-RNA interactions. In this adaptation of the RPA, RNase H is used to cleave a target RNA molecule at a specific site hybridized with a DNA probe. If a protein is bound to the RNA at the target sequence, it will prevent will block probe hybridization, prevent cleavage by RNase H and indicate a site of interaction between protein and RNA. RNase H requires only a four basepair hybrid with a DNA probe in order to cleave the RNA molecule of interest. Using many small probes allows the entire sequence of RNA to be mapped for sites of interaction.

The interactions between peptides and proteins, respectively, can be investigated by various methods, which include, but are not limited to, protein binding microarray, antibody microarrays, protein chips, and a variety of assays, UV-crosslink experiments.

The interactions between nucleic acids can be checked for instance by hybridization, which is based on the annealing of complementary DNA-DNA or DNA-RNA or RNA-RNA-sequences. The nucleotide sequences encoding GRK5 may be labeled by standard methods and added to a sample of nucleic acids to be used as test compounds under conditions suitable for the formation of hybridization complexes. After a suitable incubation period, the sample is washed and the signal is quantified and compared with a standard value. If the amount of signal in the patient sample is significantly altered from that of a comparable control sample, the nucleotide sequences have hybridized with nucleotide sequences in the sample, and the presence of altered levels of nucleotide sequences encoding GRK5 in the sample indicates the presence of the associated disorder. Such assays may also be used to evaluate the efficacy of a particular therapeutic treatment regimen in animal studies, in clinical trials, or in monitoring the treatment of an individual patient. Interactions between nucleic acids can also be investigated by microarrays. A further way of testing the binding between nucleic acids is the use of gel shift assays, in which hybrid molecules are moving slower in a denaturating gels in electrophoresis.

In all methods to identify compounds that modulate (stimulate or inhibit) the expression and kinase activity of the polypeptides of the invention, the expression level and kinase activity are compared to those detected in the absence of the test compound. The present invention is related particularly to the identification of compounds which have inhibitory activity on the kinase activity of the polypeptides of the invention. Consequently, it is particularly the inhibition of expression and activity that is measured.

The inhibition of nucleic acids on the mRNA-level encoding the polypeptides can be checked by investigating the expression of the polypeptides by quantitative methods, e.g. Western blot or enzyme-linked immune-adsorbent assay (ELISA). A way to quantify the protein expression is further the measuring of fusion proteins, wherein the polypeptides of the invention are fused to proteins or protein fragments which are easy to quantify, like fluorescent proteins. The inhibition of DNA and thus the production of mRNA can be checked by mRNA-quantification. Levels of mRNA can be quantitatively measured by Northern blotting. Another way is the reverse transcription quantitative polymerase chain reaction (RT-PCR followed by qPCR). Another way of quantifying mRNA is the use of microarrays, which are, however, more practical if a large set of mRNAs is investigated.

The inhibition of the polypeptides on the protein-level can be investigated by measuring their activity. The determination of the activity of a polypeptide/protein/enzyme depends on its specificity. Consequently, the activity of kinases is measured in phosphorylation assays, wherein a substrate is phosphorylated by a kinase. The kinase activity of GRK5 can be detected, for example, by adding ATP having radioactively labeled phosphate to the system containing the polypeptide GRK5 and the substrate and measuring the radioactivity of the phosphate attached to the substrate.

According to the invention, the effect of a test compound on the kinase activity of the polypeptide can be estimated in a system using an insulin-producing cell line, or primary cells, which are or are derived from pancreatic cells. Therein the change of the insulin release level compared to the level without the compound. The release level of insulin can be estimated with the mRNA and protein quantification levels identified above.

Polypeptide of GRK5 can be used in high-throughput screens to assay test compounds for the ability to modulate the kinase activity. These compounds can be further screened against a functional kinase to determine the effect of the compound on the kinase activity. Further, these compounds can be tested in animal or invertebrate systems to determine activity/effectiveness. Compounds can be identified that activate (agonist) or inactivate (antagonist) the kinase to a desired degree. Further, GRK5 can be used to screen a compound for the ability to stimulate or inhibit interaction between the kinase protein and a molecule that normally interacts with the kinase protein, e.g. a substrate or a component of the signal pathway that the kinase protein normally interacts (for example, another kinase). Such assays typically include the steps of combining the kinase protein with a candidate compound under conditions that allow the kinase protein, or fragment, to interact with the target molecule, and to detect the formation of a complex between the protein and the target or to detect the biochemical consequence of the interaction with the kinase protein and the target, such as any of the associated effects of signal transduction such as protein phosphorylation, cAMP turnover, and adenylate cyclase activation, etc..

Polypeptide of GRK5 is also useful in competition binding assays in methods designed to discover compounds that interact with the kinase (e.g. binding partners and/or ligands). Thus, a compound is exposed to a kinase polypeptide under conditions that allow the compound to bind or to otherwise interact with the polypeptide. Soluble kinase polypeptide is also added to the mixture. If the test compound interacts with the soluble kinase polypeptide, it decreases the amount of complex formed or activity from the kinase target. This type of assay is particularly useful in cases in which compounds are sought that interact with specific regions of the kinase.

To facilitate the identification of modulators of the expression and activity of GRK5, the invention further provides, in a preferred embodiment, a kit for a method to screen for a modulator for use in the treatment of a disease of the carbohydrate metabolism comprising
a) the GRK5 polypeptide,
   and
b) the nucleic acid encoding GRK5
   and
c) a control compound known to affect the insulin production by binding the GRK5 polypeptide or the corresponding nucleic acid,
   and further comprising
d) a cell line with insulin production.

In a further preferred embodiment, the invention provides a kit for a method to screen for a modulator for use in the treatment of a disease of the carbohydrate metabolism comprising
a) GRK5 polypeptide,
   and
b) a nucleic acid encoding GRK5 ,
   and
c) a control compound for the kinase GRK5 known to affect the insulin production by binding the GRK5 polypeptide or the corresponding nucleic acid,
   and further comprising
d) a cell line with insulin production.

In all embodiments of the kit, the control compounds can be any of the test compounds characterized above. A control compound is used as a reference for the binding/inhibitory efficiency of a test compound because it is known for its binding to a chosen polypeptide or the corresponding nucleic acid which encode the chosen polypeptide, thereby inhibiting the activity or the expression of the polypeptides. A chosen control compound refers to the same polypeptide for which inhibitory compounds are tested
Control compounds that affect the insulin release by binding to the polypeptide of GRK5 are e.g. Sunitinib and siRNAs described herein or any other compound which has proved to modulate the insulin production.

An inventive compound known to affect the expression and/or activity of the polypeptide of GRK5 can be used for the treatment of diabetes mellitus, more preferably diabetes mellitus type 2, and most preferably for increasing the level of insulin release from pancreas cells by administration of the inventive compound(s) within pharmaceutical compositions as outline above. In a preferred embodiment of the invention, at least SCYL1 or ADCK1 and GRK5 are modulated by one or more of the inventive compounds.

The inventive compounds or inventive compositions are according to the invention useful for each single disease of the group of diseases consisting of diseases of the carbohydrate metabolism.

The influence of the kinase GRK5 on insulin release suggests a particular, but not limited to, utilization of the polypeptide for diagnosis of disease of the carbohydrate metabolism, preferably of diabetes mellitus, more preferably of diabetes mellitus type 2.

The embodiments in the description and the following examples are provided by way of illustration of the invention and are not included for the purpose of limiting the invention. The variations and changes of the invention which are obvious to a person skilled in the field and solutions equivalent to embodiments described herein fall within the scope of protection of the patent claims.

**Table 1 Sequence identities of the target genes and target proteins**

| **SeqIdNo** | **Sequence Name** | **Gene Accession** |
|---|---|---|
| 8 | SCYL1 gene | NM_023912 |
| 9 | GRK5 gene | NM_018869 |
| 10 | ADCK1 gene | MM_028105 |
| 11 | SCYL1 protein | NM_023912 |
| 12 | GRK5 protein | NM_018869 |
| 13 | ADCK1 protein | NM_028105 |

### Examples

### Example 1: siRNA screen in the pancreatic beta cell line beta-TC6:

To identify the kinases, which might be responsible for the elevated insulin release after Sunitinib treatment, we performed a kinome wide siRNA knock-down screen. The effect on the insulin release of each kinase depletion was monitored by using a rat/mouse insulin ELISA. The resulting data were compared to Sunitinib treatment (5 µM) as positive control and correlated to a non-targeting siRNA. Candidate genes were limited by using hierarchical clustering and by proposing a significant in-/decrease of the insulin release by 15%. Depletion of SCY1-like 1 (SCYL1), aarF-containing kinase 1 (ADCK1), and G protein-coupled receptor kinase 5 (GRK5), resulted in an increase of the insulin release in beta-TC6 cells compared to the control siRNA, rendering those kinases as potential negative modulators of insulin release (Fig. 1).

### Example 2: Validation of the negative modulators

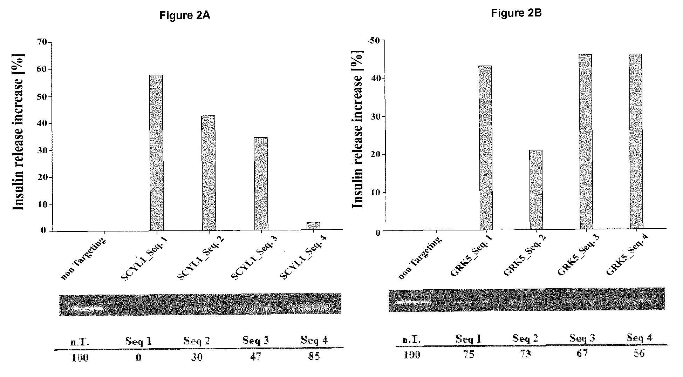

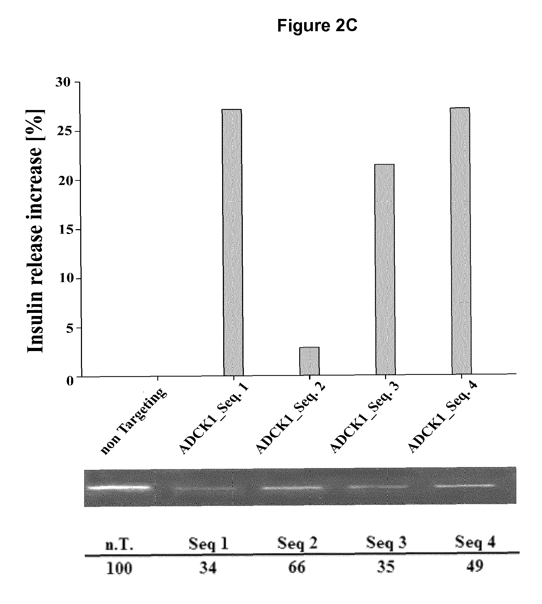

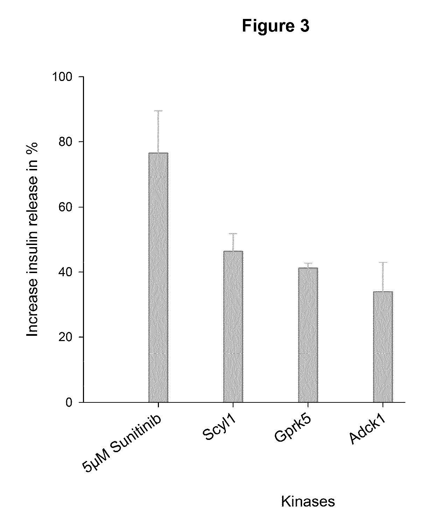

For the validation of the negative modulating kinases SCYL1, GRK5, and ADCK1, , the target genes were depleted using four different siRNA-sequences each (Fig. 2A-D). The gene-depletion was measured via mRNA-levels after 72 hours while the insulin release was measured using a rat/mouse insulin ELISA after two hours incubation. The depletion of the residual kinases SCYL1, GRK5, and ADCK1 led to an increased insulin release with different efficiencies. Furthermore, the insulin release for the kinases inversely correlated with the respective knock-down efficiency of SCYL1 and ADCK1 which ranged from 50 to nearly 100 % as estimated by RT-PCR and scanning densitometry. In case of GRK5 where all sequences lead to equal knock-down efficiency, this correlation could not be observed. The increase was highest for the depletion of SCYL1 (46,38 ± 5,51 %), followed by GRK5 (41,23 ± 1,53 %), and ADCK1 (33,95 ± 9,02 %). Sunitinib was included as a control (Fig. 3). This enhances the role of those kinases in triggering the insulin release.

**Table 2 Sequences of the primers used in RT-PCR for target validation**

| **SeqIdNo** | **GeneSymbol** | **Primer** | **GeneAccession** | **Sequence 5' - 3'** |
|---|---|---|---|---|
| 1 | SCYL1 | Fwd | NM_023912 | CGGCGGCGACGATGTGGTTCTTT |
| 2 | SCYL1 | Rev | NM_023912 | CGGCGTTGCCCTGTGCCGAGTA |
| 3 | ADCK1 | Fwd | NM_028105 | CTGACACGGGCAAGGCTGAGATT |
| 4 | ADCK1 | Rev | NM_028105 | GCGCCCTGATACAACACCGAGAC |
| 5 | GRK5 | Fwd | NM_018869 | GCCGGGTGCTGGAGACTGAGGA |
| 6 | GRK5 | Rev | MM_018869 | TGGCGGTTCTGGAGGCTGACTTCT |

### Example 3: Results double knock-down of the validated kinase

Compared to the Sunitinib treatment, the effect of the single knock-downs was less pronounced suggesting the involvement of multiple kinases in triggering the insulin release. The candidate kinases are widespread in various signaling pathways. To investigate whether the kinases have a redundant or additive effect on insulin release, we performed double-knock-downs for each possible kinase pair. The insulin increase due to the double knock-down was correlated to the single knock-downs as well as to the non-targeting siRNA. Out of 16 kinase pairs, SCYL1 and ADCK1 depletion resulted in the highest insulin release (90,64 ± 17,32 %), which was equal to the Sunitinib induced insulin release (Fig. 4). For the other kinase pairs, no increased insulin release compared to the single-knock-downs was observed (data not shown).

### Example 4: Phosphorylation of AKT1 upon reduction of kinase C candidate gene expression in beta-TC6 cells

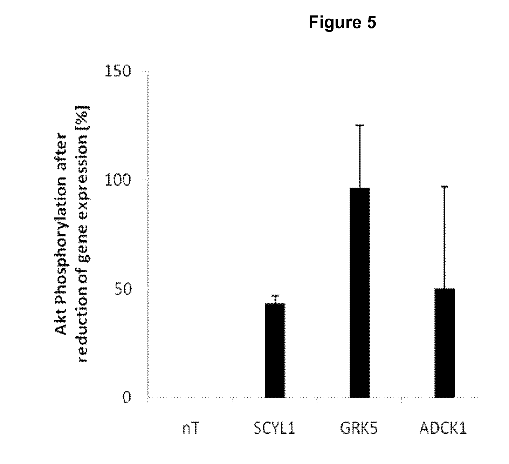

The gene expression of SCYL1, GRK5, and ADCK1 was inhibited by siRNA. Downregulation of SCYL1, GRK5 as well as ADCK1 increased phosphorylation of AKT with a tolerable standard deviation (SCYL1: 43,73 ± 3,37 %; GRK5: 96,53 ± 28,87 %; ADCK1:) (Fig. 5). It can be concluded that the siRNA mediated reduction of the gene expression results in increased AKT1 phosphorylation. As already mentioned, this increase seems to be connected to insulin release according to the publication by Leibiger B. and colleagues (Leibiger et al. FASEB J, 2010, 24; 1824 - 1837).

### Example 5: Measurement of SCYL1, GRK5, and ADCK1 mRNA levels upon 24h Sunitinib treatment

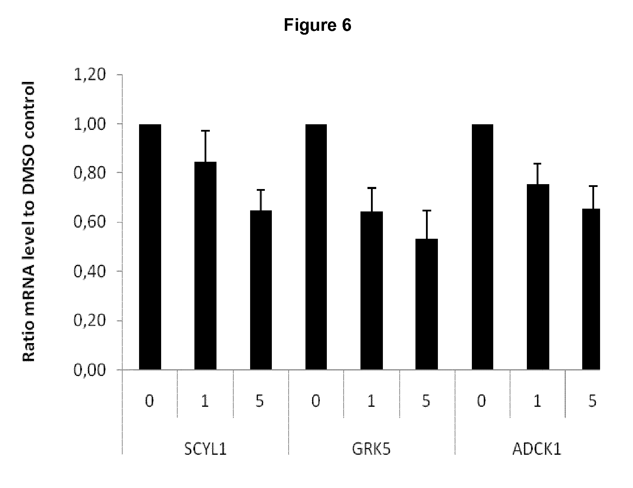

Beta TC6 cells were treated with 1 µM and 5 µM, respectively, Sunitinib for 24h. The inhibition of gene expression was estimated by measurement of mRNA levels. Sunitinib treatment negatively influences mRNA levels of candidate kinases (Figure 6). This observation might represent a further explanation for the positive effect of Sunitinib on diabetes patients in clinics where Sunitinib is applied for a longer period of time.

### Example 6: Uptake of the fluorescent glucose analogue 2-NBDG upon candidate kinase knock-down in beta C2C12 and 3T3-L1 cells

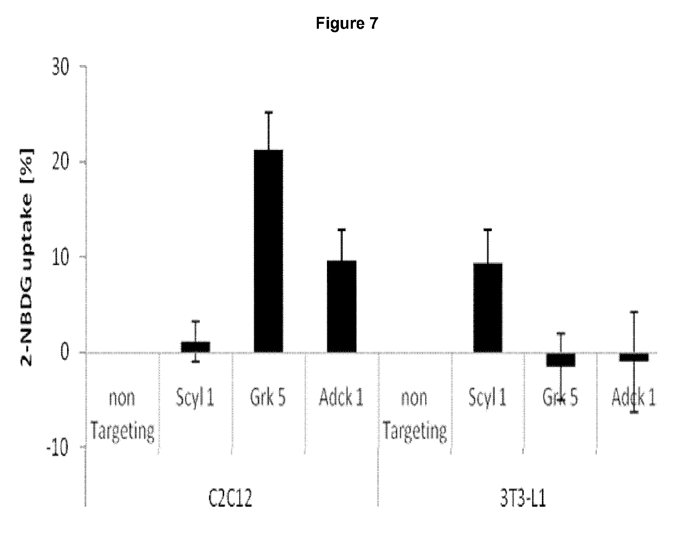

The glucose uptake of cells was investigated in response to reduction of candidate gene expression. Down-regulation of GRK5 remarkably affects uptake of 2-NBDG in C2C12 (GRK5: 21,24 ± 3,96 %) whereas ADCK1 showed an increase of 9,72 ± 3,81 %. In 3T3-L1 cells the reduction of gene expression for SCYL1 gene reduction shows an impact on glucose uptake (9,45 ± 3,45 %) (Fig. 7). All data are presented as mean values (± SEM). It can be concluded that the reduction of the G protein-coupled receptor kinase 5 (GRK5) and ADCK1 expression seems to enhance uptake of the glucose analogue 2-NBDG (21,24 ± 3,96 %) in mouse myoblast cells (C2C12) without need of insulin stimulation, thus GRK5 seems to trigger the insulin sensitivity and/or insulin independent glucose uptake.

### Example 7: Phosphorylation of AKT1 upon candidate target knock-down in C2C12 and 3T3-L1 cells

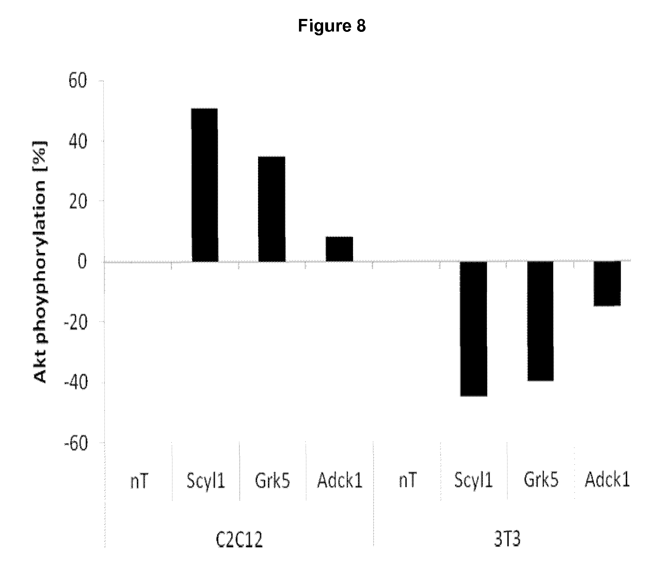

The phosphorylation of AKT1 was investigated upon the reduction of gene expression of SCYL1, GRK5, and ADCK1. As illustrated, downregulation of SCYL1 as well as GRK5 increases phosphorylation of AKT1 in C2C12 cells. In the 3T3-L1 model system we detected a decrease of AKT1 phosphorylation for all candidate kinases (Fig. 8). It can be concluded that in peripheral tissues AKT1 phosphorylation is connected to GLUT4 translocation and therefore to glucose uptake. In the beta-TC6 cell line, the knock-down of SCYL1 and GRK5 increases phosphorylation of AKT1. Furthermore, the results of the 2-NBDG uptake assay in C2C12 cells as well as the observed increase in AKT1 phosphorylation in the beta-TC6 upon reduction of candidate gene expression might explain the functional role of GRK5 in regard to an elevated release of insulin.

### Example 8: Analysis of a potential glucose mediated effect on insulin released by beta TC6 cells

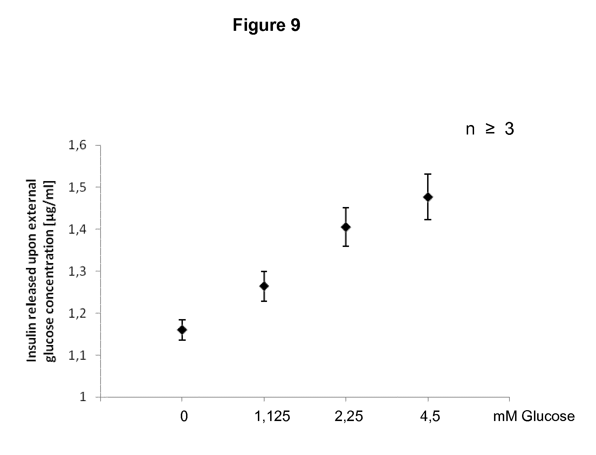

The influence of external glucose was investigated at glucose concentrations ranging from 1,125 to 4,5 mM relative to glucose free control (Fig. 9). The addition of different glucose concentrations ranging from physiological to pathophysiological concentrations (ranging from 1 g/L (5,5 mM) up to 4,5 g/L (25,5 mM)) to media of beta-TC6 cells does not results in a concentration dependent increase in insulin release. However, using concentrations under 0,8 g/L (4,5 mM) glucose displayed a glucose dependent release of insulin (Poitout et al., Diabetes, 1995, 4; 306 - 313). Thus a glucose concentration of 0,2 g/L (1,125 mM) was chosen for further experiments with a low glucose environment.

### Example 9: Insulin released by beta T6 cells after reduction of gene expression in low glucose media

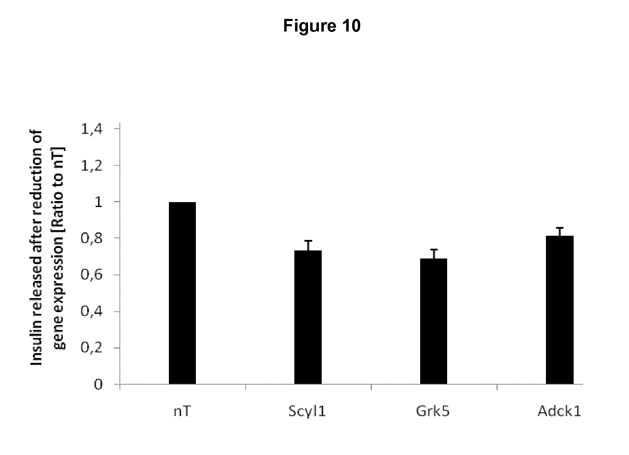

The insulin release was mediated by reduction of gene expression in a low glucose environment of 0,2 g/L (1,125 mM) (Fig. 10). It can be concluded that knock-down of SCYL1, GRK5, and ADCK1 seems to decrease the insulin release in a low glucose environment. A performed MTT-assay displayed no impaired cell viability.

### Example 10: GRK5 as anti-diabetic target for drug development

8800 Customer compounds were screened for their ability to inhibit the enzymatic activity of GRK5. Screening was performed using the ADP Glo™ Kinase Assay technology (Promega). The small molecular weight compounds were delivered at 10 mM stock concentration and were tested at a final assay concentration of 100 µM. A narrow hit distribution was observed with an average inhibition of 3.2% and a standard deviation of 11%. In consequence compounds with an inhibition value above 36.1% (= inhav + (3 x inhstdev)) are recommended to be considered as hit. The top candidate compounds are summarized in table 3. All five compounds identified by the screen have a molecular weight in the range of 200 to 450 g per mol.

**Table 3: Top candidates revealed by the screen**

| **Compound** | **Inhibition of Grk5 [%]** |
|---|---|
| **C1** | 68,0 |
| **C2** | 60,0 |
| **C3** | 56,0 |
| **C4** | 52,0 |
| **C5** | 51,0 |

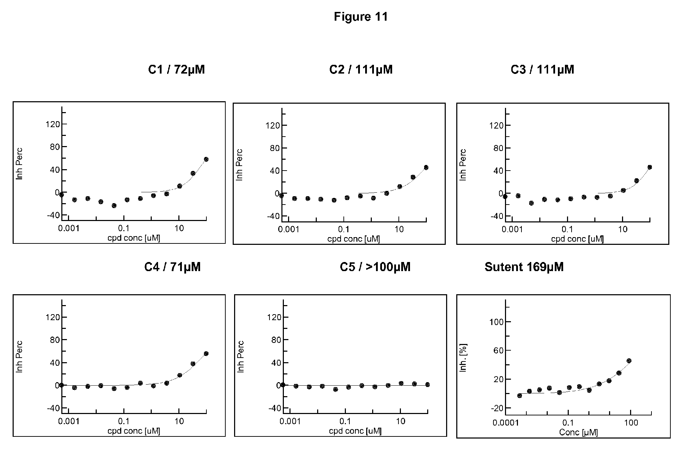

IC₅₀ values were determined in triplicates at 12 concentrations with a dilution factor of three. Triplicates were measured on three different assay plates. The maximal compound assay concentration was set to 100 µM representing the compound assay concentration used in the primary screen. A larger maximal compound assay concentration could not be achieved since the maximal compound concentration on the compound plates was 10 mM (>1% DMSO concentration in the assay). For all assay plates z prime values were found to be significantly larger than 0.5 proving statistical relevance of the data (see Table 4). Since the majority of all primary screening hits showed less than 50% GRK5 inhibition at 100 µM primary screening concentration, also the majority of all IC₅₀ graphs did not reach 50% inhibition at the maximal compound concentration of 100 µM. For these compounds the IC₅₀ values were extrapolated if the at least 30% GRK5 inhibition was observed at the maximal compound concentration of 100 µM. For 62% of the examined compounds a valid IC₅₀ value could be determined. The remaining 38% of the compounds had less than 30% inhibition at the highest compound concentration of 100 µM (Fig. 11, Table 4).

**Table 4: IC50 values of the 5 top candidates**

| **Copound_ID** | **IC50 [µM]** | **ZPRIME (assay plate 1/2/3)** |
|---|---|---|
| C1 | 72 | 0.87/0.90/0.92 |
| C2 | 111 | 0.87/0.90/0.92 |
| C3 | 111 | 0.87/0.90/0.92 |
| C4 | 71 | 0.87/0.90/0.92 |
| C5 | >100 | 0.87/0.90/0.92 |
| Sutent | 169 | 0.87/0.90/0.92 |

### Example 11: Release of Insulin after inhibition of GRK5 by compounds 1 - 5 in a beta TC6-cell system

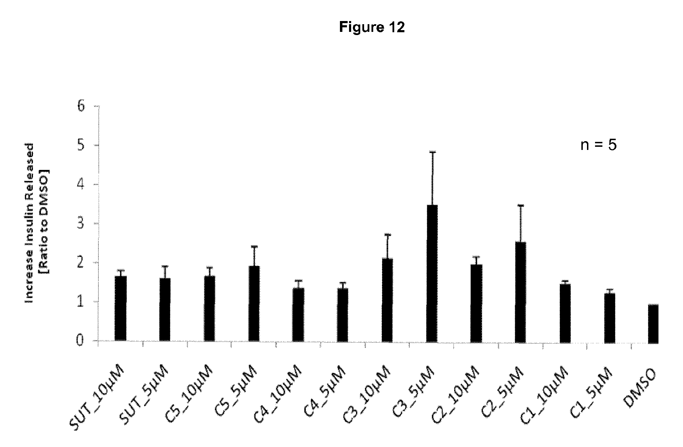

Insulin release in beta-TC6 cells was increased by GRK5 inhibitor treatment. The inhibition by compound two (C2), three (C3) and five (C5) led to the most remarkable effect of elevating insulin release by doubling the effect of Sunitinib (SUT) in our system (C2: 2,58 ± 0,94 at 5µM and 2,01 ± 0,19 at 10µM; C3: 3,52 ± 1,35 at 5µM and 2,15 ± 0,59 at 10µM; C5: 1,93 ± 0,5 at 5µM and 1,69 ± 0,19 at 10µM) (Fig. 12). The values are also displayed in table 5.

**Table 5: Averages and SEM of Figure 12**

| **SUT_10µM** | **SUT_5µM** | **C5_10µM** | **C5_5µM** | **C4_10µM** | **C4_5µM** | |
|---|---|---|---|---|---|---|
| 1,65 | 1,61 | 1,69 | 1,93 | 1,38 | 1,37 | |
| 0,14 | 0,31 | 0,19 | 0,50 | 0,18 | 0,14 | |
| | | | | | | |

| **C3_10µM** | **C3_5µM** | **C2_10µM** | **C2_5µM** | **C1_10µM** | **C1_5µM** | **DMSO** |
|---|---|---|---|---|---|---|
| 2,15 | 3,52 | 2,01 | 2,58 | 1,52 | 1,29 | 1 |
| 0,59 | 1,35 | 0,19 | 0,94 | 0,06 | 0,08 | 0 |

It was shown that all of the GRK5-screen based inhibitors stimulate insulin release in the beta TC6 cell system. Thus the target GRK5 has been validated as important regulator of insulin secretion. Furthermore, the inhibition by compounds two, three and five led to the most remarkable effect of elevating insulin release by doubling the effect of Sunitinib in our system (C2: 2,58 ± 0,94 at 5µM and 2,01 ± 0,19 at 10µM; C3: 3,52 ± 1,35 at 5µM and 2,15 ± 0,59 at 10µM; C5: 1,93 ± 0,5 at 5µM and 1,69 ± 0,19 at 10µM).

### Example 12: Insulin released after blocking of protein biosynthesis with cycloheximid and GRK5 inhibitor treatment

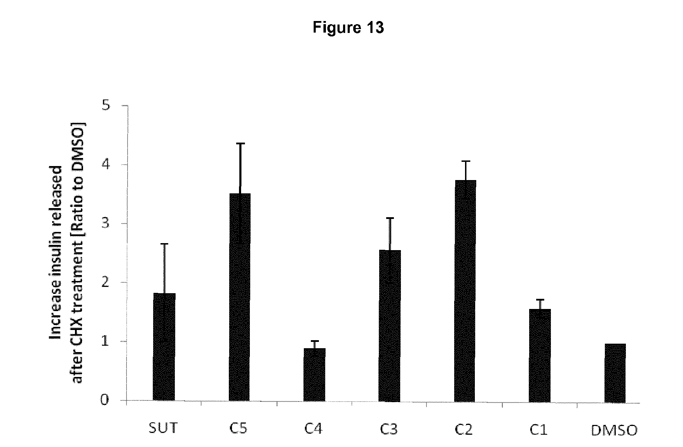

It was found that the impact of GRK5 inhibitor on insulin release is not based on intensified insulin synthesis. The insulin release was measured upon cycloheximid (CHX) and appropriate 5µM compound treatment compared to control treated with cycloheximid and DMSO in mean values (± SEM) (Fig.13). It can be concluded that blocking of the protein transcription and thus of renewing insulin by protein biosynthesis does not affect insulin release upon exposure to the GRK5 inhibitor compounds.

### Example 13: Phosphorylation of AKT1 in beta-TC6 cells by GRK5 inhibitor

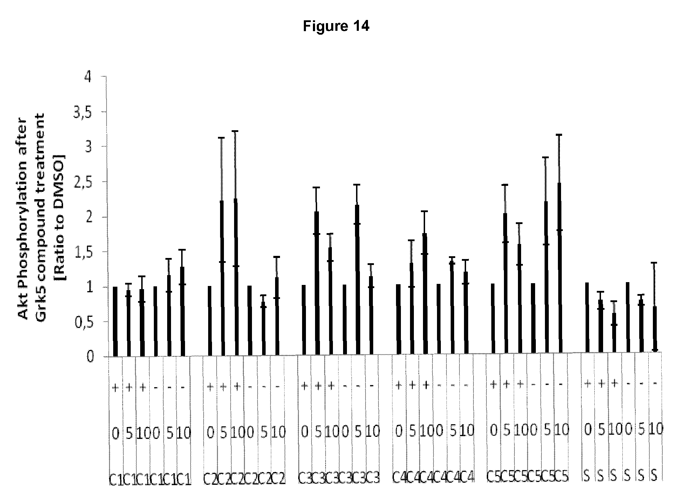

The phosphorylation of AKT1 was measured upon GRK5 inhibitor treatment. The beta-TC6 cells were treated by 5µM respectively 10µM of appropriate compound and compared to a DMSO control as well as to AKT protein levels. It can be concluded that AKT1 is phosphorylated by a 1 µg/mL insulin treatment and on that the phosphorylation of Akt1 is elevated by treatment with GRK5 inhibitor (Fig. 14). In contrast to this, Sunitinib - as a control - decrease the insulin mediated increase in AKT1 phosphorylation.

Together with previously described knock down studies for AKT phosphorylation and 2-NBDG uptake we suggest, that GRK5 plays a role in glucose uptake and thus leads to increased insulin release.

### Example 14: Insulin released by beta TC6 cells after inhibition of GRK5 in low glucose environment (1.25 mM)

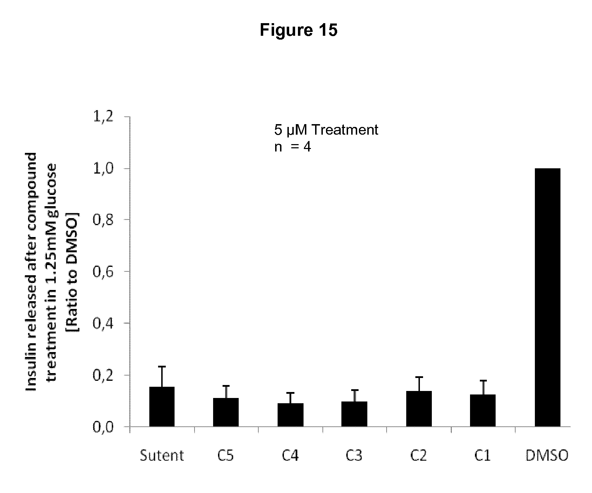

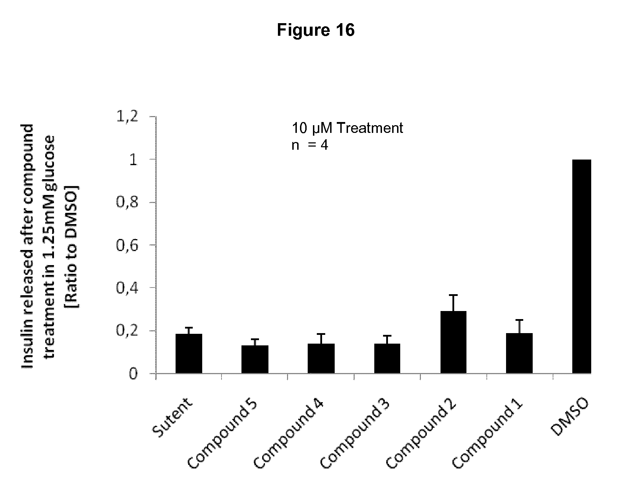

The inhibition of GRK5 in a low glucose environment led to a decreased insulin secretion after a 5 µM treatment (Fig. 15) and 10 µM treatment (Fig. 16). It can be concluded that the inhibition of GRK5 by 5 µM respectively 10 µM of the revealed compounds led to a decrease of insulin release in a low glucose media (1,125 mM). A performed MTT-assay displayed no impaired cell viability.

### Example 15: Glucose uptake via 2-NBDG after GRK5 compound treatment in C2C12, TC6, 3T3-L1 and cells

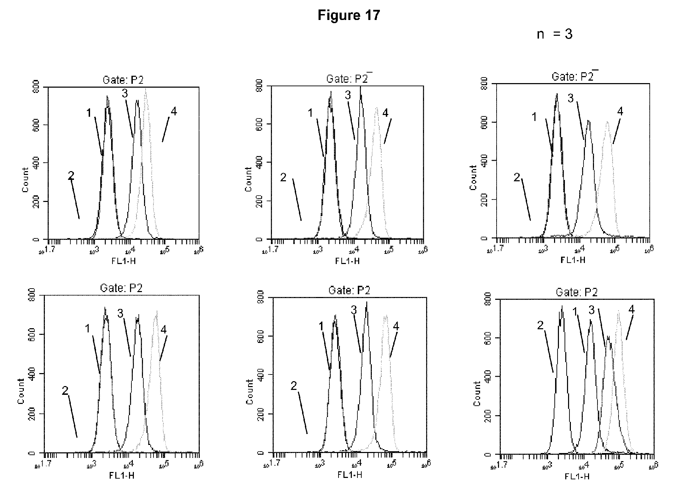

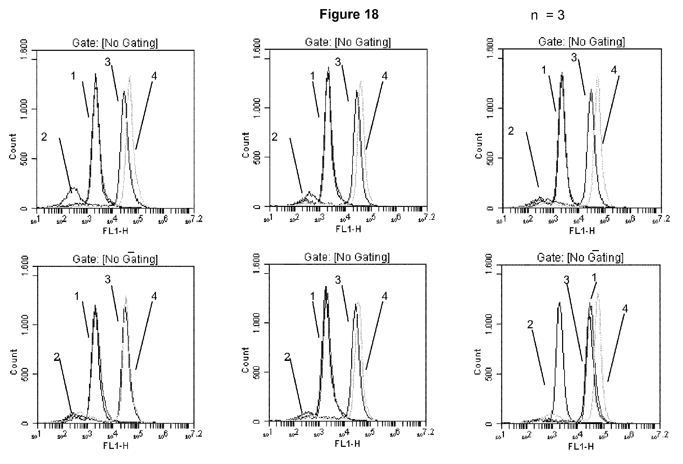

Inhibition of GRK5 led to an increased uptake of the fluorescent glucose analogue 2-NBDG in C2C12 (Fig. 17) and TC6 (Fig. 18) cells for all five compounds. We suggest that inhibition of GRK5 leads to increased glucose sensitivity without need of insulin. Thus blocking of GRK5 phosphorylation could substitute for insulin.

The increase of glucose uptake in beta TC6 cells incline with previously observed AKT phosphorylation and insulin release. Somehow, the glucose uptake in 3T3-L1 pre-adipocytes (Fig. 18) could only be stimulated by compound 2, suggesting that the cell model is probably not adequate or should be differentiated.

The data are summarized in Table 6:

**Table 6: Averages and SEM of Figure 17 - 19**

| | **C2C12** | | **beta TC6** | | **3T3-L1** | | *n* = 3 |
|---|---|---|---|---|---|---|---|
| | **Ratio** | **SE** | **Ratio** | **SE** | **Ratio** | **SE** | |
| **DMSO** | 1 | 0 | 1 | 0 | 1 | 0 | |
| **C1** | 2,346 | 0,53 | 1,567 | 0,12 | 0,989 | 0,07 | |
| **C2** | 2,127 | 0,44 | 1,529 | 0,07 | 1,111 | 0,03 | |
| **C3** | 2,325 | 0,46 | 1,683 | 0,21 | 1,022 | 0,03 | |
| **C4** | 1,774 | 0,43 | 1,284 | 0,09 | 0,930 | 0,07 | |
| **C5** | 1,47 | 0,17 | 1,259 | 0,02 | 0,926 | 0,04 | |
| **Sut** | 1,511 | 0,4 | 1,084 | 0,19 | 0,533 | 0,10 | |

### Example 16: Glucose uptake via 2-NBDG after GRK5 compound treatment in matured C2C12 myotubes and 3T3-L1

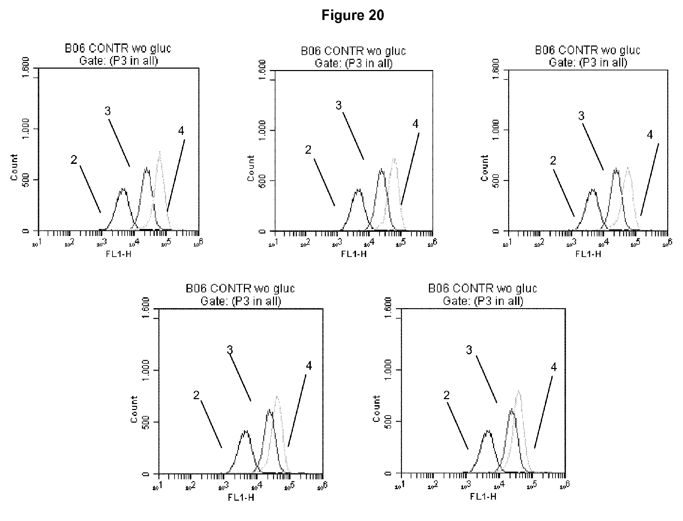

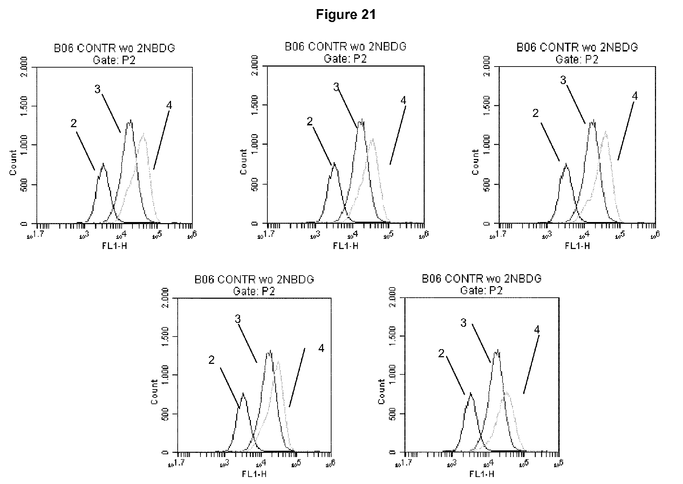

Inhibition of GRK5 led to an increased uptake of the fluorescent glucose analogue 2-NBDG in matured C2C12 myotubes (Fig. 20) and 3T3-L1 adipocytes (Fig. 21) for all five compounds (summarized in table 4). In line with C2C12 cells, the matured myotubes display an increase 2-NBDG uptake after inhibition of GRK5 by compounds 1-5. Moreover, also matured adipocytes respond to all five compounds suggesting that glucose metabolism for adipocytes is basically higher and thus could be detected easily.

**Table 7: Averages and SEM of Figure 20 and 21**

| | **C2C12 matured myotubes** | | **3T3-L1 matured adipocytes** | | *n* = 2 |
|---|---|---|---|---|---|
| | **Ratio** | **SE** | **Ratio** | **SE** | |
| **DMSO** | 1 | 0 | 1 | 0 | |
| **C1** | 1,976 | 0,101 | 2,520 | 0,071 | |
| **C2** | 2,383 | 0,637 | 2,234 | 0,085 | |
| **C3** | 2,457 | 0,567 | 2,141 | 0,031 | |
| **C4** | 1,829 | 0,396 | 1,583 | 0,043 | |
| **C5** | 2,033 | 0,210 | 1,491 | 0,043 | |

### Description of the figures

- Figure 1:: Assortment of the positive and negative regulating kinases of the kinome screen. The candidate kinases were limited by using hierarchical clustering and proposing and increased insulin release of 15 % significant. Additionally, the data were correlated to Sunitinib treatment (5 µM) and a non-targeting siRNA as positive and negative controls. The results are shown for n ≥ 4 biological independent experiments.
- Figure 2:: Correlation of the insulin release increase to the target depletion for each used siRNA sequence for the kinases SCYL1 (Fig. 2A), GRK5 (Fig. 2B), and ADCK1 (Fig. 2C). The insulin release for the kinases (bar chart; upper panel) correlated with their knock-down efficiency, which was monitored by RT-PCR and scanning densitometry (agarose gel; middle panel; Δ depletion; lower panel). The insulin increase as well as the knock-down efficiency is compared to the insulin release or gene depletion of the non-targeting siRNA.
- Figure 3:: Validation of insulin release increase for kinases SCYL1, GRK5 and ADCK1. SCYL1 showed the most reliable increase in insulin release after gene-depletion with about 46,38 ± 5,51 %, followed by GRK5 (41,23 ± 1,53 %), and ADCK1 (33,95 ± 9,02 %). The control Sunitinib resulted in 76,52 ± 13 %.
- Figure 4:: Additional increase of the insulin release due to a double knock-down for the kinase pair SCYL1 and ADCK1. The depletion resulted in the highest Insulin release (90,64 ± 17,32 %), which equaled the insulin after Sunitinib treatment. The figure depicts the values of the single knock-down (light grey and grey), the theoretical mathematical value (changeover dark grey to grey) and the real insulin increase after a double knock-down (dark grey).
- Figure 5:: Phosphorylation of AKT1 upon reduction of gene expression of SCYL1, GRK5 and ADCK1.
- Figure 6:: Measurement of SCYL1, GRK5 and ADCK1 mRNA levels upon Sunitinib treatment of 24 h.
- Figure 7:: Uptake of the fluorescent glucose analogue 2-NBDG upon candidate kinase gene knock-down in beta C2C12 and 3T3-L1 cells.
- Figure 8:: Phosphorylation of AKT1 upon candidate target knock-down in C2C12 and 3T3-L1 cells.
- Figure 9:: Analysis of a potential glucose-mediated effect on insulin released by beta TC6 cells (1.125 to 4.5 mM Glucose).
- Figure 10:: Insulin released by beta TC6 cells after reduction of gene expression in low glucose media.
- Figure 11:: IC₅₀ graphs of GRK5 primary hit compounds.
- Figure 12:: Release of Insulin after inhibition of GRK5 by compounds 1 - 5 in a beta TC6-cell system.
- Figure 13:: Insulin released after blocking of protein biosynthesis with cycloheximid and GRK5 inhibitor treatment.
- Figure 14:: Phosphorylation of AKT in beta-TC6 cells by GRK5 Inhibitor.
- Figure 15:: Insulin released by beta TC6 cells after inhibition of GRK5 in low glucose environment (1.25 mM) with 5 µM compound.
- Figure 16: Insulin released by beta TC6 cells after inhibition of GRK5 in low glucose environment (1.25 mM) with 10 µM compound.
- Legend Figure 17 - 21:: 1 = wo 2-NBDG + compound (glucose free medium + compound);
2 = wo 2-NBDG (glucose free medium); 3 = 100µM 2-NBDG
4 = 100µM 2-NBDG + compound
Upper panel left to right shows always compounds 1 - 3, lower panel left to right compounds 4 - 5 and Sunitinib. (Figure 20 and 21: Sunitinib is missed9
- Figure 17:: Uptake of glucose analogue 2-NBDG after inhibition of GRK5 in C2C12 mouse myoblasts.
- Figure 18:: Uptake of glucose analogue 2-NBDG after inhibition of GRK5 in beta TC6 cells.

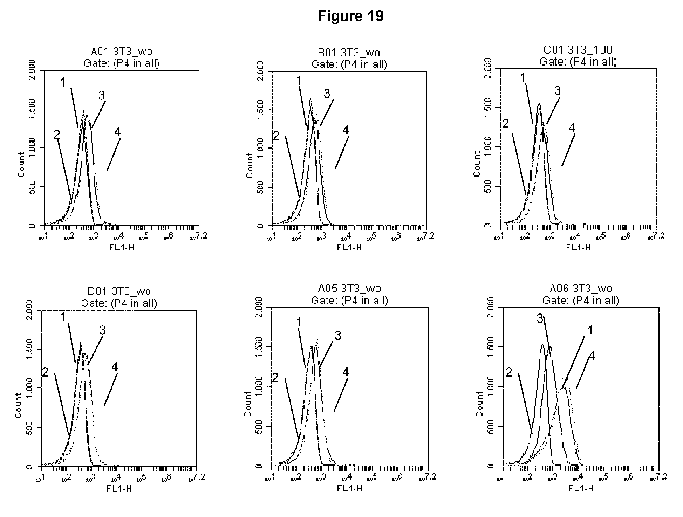

- Figure 19:: Uptake of glucose analogue 2-NBDG after inhibition of GRK5 in beta 3T3-L1 cells.
- Figure 20:: Uptake of glucose analogue 2-NBDG after inhibition of GRK5 in matured C2C12 myotubes.
- Figure 21:: Uptake of glucose analogue 2-NBDG after inhibition of GRK5 in 3T3-L1 adipocytes.

## Claims

1. A modulator for
a) inhibition of the protein kinase GRK5 or
b) inactivation, degradation, downregulation, or intercalation of the nucleic acid encoding GRK5,
for use in the treatment of a disease of the carbohydrate metabolism,
wherein the modulator is a small molecule, an siRNA molecule, an antisense oligonucleotide and wherein the modulator is not sunitinib.

2. The modulator for use according to claim 1, wherein the disease is diabetes.

3. The modulator for use according to claim 1 or 2, wherein the disease is diabetes mellitus type 2.

4. The modulator for use according to claim 1, 2 or 3, wherein said modulator is used for the up-regulation of insulin production and/or release of insulin.

5. A pharmaceutical composition comprising the modulator of claim 1 for use in the treatment of a disease of the carbohydrate metabolism.

6. A pharmaceutical composition comprising the modulator of claim 1 for use according to claim 5, wherein the disease is diabetes.

7. A pharmaceutical composition comprising the modulator of claim 1 for use according to claim 5 or 6, wherein the disease is diabetes mellitus type 2.

8. A pharmaceutical composition comprising the modulator of claim 1 for use according to claim 5, 6 or 7 used for the up-regulation of insulin production and/or release of insulin.

9. A method for screening for a modulator for use in the treatment of a disease of the carbohydrate metabolism, the method comprising
a) contacting a test compound with the polypeptide of GRK5,
b) detecting the binding of said test compound to the GRK5 polypeptide, and
c) determining the activity of the GRK5 polypeptide in the presence of said test compound,
wherein step c) comprises determining a change of the insulin release level compared to the level without the test compound.

10. The method according to claim 9, wherein instead of polypeptides nucleic acids encoding the polypeptides are used and the expression rate is determined instead of the activity.

11. The method according to claim 9 or 10, wherein the test compound is RNA or a peptide or an antibody or a kinase inhibitor.

12. A kit for a method to screen for a modulator for use in the treatment of a disease of the carbohydrate metabolism comprising
a) GRK5 polypeptide,
b) a nucleic acid encoding GRK5,
c) a cell line with insulin production,
d) a control compound known to affect the insulin production by binding the GRK5 polypeptide or the corresponding nucleic acid.

## Patentansprüche

1. Ein Modulator zur
(a) Inhibierung der Proteinkinase GRK5 oder
(b) Inaktivierung, Abbau, Herunterregulierung oder Interkalation der Nukleinsäure, die GRK5 codiert,
zur Verwendung in der Behandlung einer Krankheit des Kohlenhydratstoffwechsels,
wobei der Modulator ein kleines Molekül, ein siRNA-Molekül, ein Antisense-Oligonukleotid ist und wobei der Modulator nicht Sunitinib ist.

2. Der Modulator zur Verwendung gemäß Anspruch 1, wobei die Krankheit Diabetes ist.

3. Der Modulator zur Verwendung gemäß Anspruch 1 oder 2, wobei die Krankheit Diabetes mellitus Typ 2 ist.

4. Der Modulator zur Verwendung gemäß Anspruch 1, 2 oder 3, wobei besagter Modulator zur Hochregulierung der Insulin-Produktion und/oder Freisetzung von Insulin verwendet wird.

5. Eine pharmazeutische Zusammensetzung, umfassend den Modulator von Anspruch 1 zur Verwendung in der Behandlung einer Krankheit des Kohlenhydratstoffwechsels.

6. Eine pharmazeutische Zusammensetzung, umfassend den Modulator von Anspruch 1 zur Verwendung gemäß Anspruch 5, wobei die Krankheit Diabetes ist.

7. Eine pharmazeutische Zusammensetzung, umfassend den Modulator von Anspruch 1 zur Verwendung gemäß Anspruch 5 oder 6, wobei die Krankheit Diabetes mellitus Typ 2 ist.

8. Eine pharmazeutische Zusammensetzung, umfassend den Modulator von Anspruch 1 zur Verwendung gemäß Anspruch 5, 6 oder 7, der für die Hochregulierung der Insulin-Produktion und/oder Freisetzung von Insulin verwendet wird.

9. Ein Verfahren zum Screening eines Modulators zur Verwendung in der Behandlung einer Krankheit des Kohlenhydratstoffwechsels, die Methode umfassend
a) Kontaktieren einer Testverbindung mit dem Polypeptid von GRK5,
b) Erkennen der Bindung der besagten Testverbindung an das GRK5-Polypeptid, und
c) Bestimmen der Aktivität des GRK5-Polypeptids in Anwesenheit von besagter Testverbindung,
wobei Schritt c) Bestimmen einer Änderung des Insulinfreisetzungslevels im Vergleich zu dem Level ohne die Testverbindung umfasst.

10. Das Verfahren gemäß Anspruch 9, wobei anstelle von Polypeptiden Nukleinsäuren, die Polypeptide kodieren verwendet werden und die Expressionsrate statt der Aktivität bestimmt wird.

11. Das Verfahren gemäß Anspruch 9 oder 10, wobei die Testverbindung RNA oder ein Peptid oder ein Antikörper oder ein Kinase-Inhibitor ist.

12. Ein Kit für ein Verfahren zum Screenen eines Modulators zur Verwendung in der Behandlung einer Krankheit des Kohlenhydratstoffwechsels, umfassend
a) GRK5-Polypeptid,
b) eine Nukleinsäure, die GRK5 kodiert,
c) eine Zell-Linie mit Insulin-Produktion,
d) eine Kontrollverbindung, die bekannt ist die Insulin-Produktion durch Binden des GRK5-Polypeptids oder der entsprechenden Nukleinsäure zu beeinflussen.

## Revendications

1. Un modulateur pour
a) l'inhibition de la protéine kinase GRK5, ou
b) l'inactivation, la dégradation, la régulation négative ou l'intercalation de l'acide nucléique codant pour GRK5,
destiné à être utilisé dans le traitement d'une maladie du métabolisme des glucides,
où le modulateur est une petite molécule, une molécule d'ARNsi, un oligonucléotide antisens, et où le modulateur n'est pas sunitinib.

2. Le modulateur destiné à être utilisé selon la revendication 1, où la maladie est le diabète.

3. Le modulateur destiné à être utilisé selon la revendication 1 ou 2, où la maladie est le diabète sucré de type 2.

4. Le modulateur destiné à être utilisé selon la revendication 1, 2 ou 3, où ledit modulateur est utilisé pour la régulation positive de la production de l'insuline et/ou la libération de l'insuline.

5. Une composition pharmaceutique comprenant le modulateur selon la revendication 1 destinée à être utilisée dans le traitement d'une maladie du métabolisme des glucides.

6. La composition pharmaceutique comprenant le modulateur selon la revendication 1 destinée à être utilisée selon la revendication 5, où la maladie est le diabète.

7. La composition pharmaceutique comprenant le modulateur selon la revendication 1 destinée à être utilisée selon la revendication 5 ou 6, où la maladie est le diabète sucré de type 2.

8. La composition pharmaceutique comprenant le modulateur selon la revendication 1 destinée à être utilisée selon la revendication 5, 6 ou 7, utilisée pour la régulation positive de la production de l'insuline et/ou la libération de l'insuline.

9. Un procédé de criblage d'un modulateur destiné à être utilisé pour le traitement d'une maladie du métabolisme des glucides, le procédé comprenant :
a) mettre en contact un composé d'essai avec le polypeptide de GRK5,
b) détection de la liaison dudit composé d'essai à la polypeptide GRK5, et
c) détermination de l'activité du polypeptide GRK5 en présence dudit composé d'essai,
où l'étape c) comprend la détermination d'un changement du niveau de l'insuline libérée comparé au niveau sans le composé d'essai.

10. Le procédé selon la revendication 9, dans lequel des acides nucléiques codant pour les polypeptides sont utilisés au lieu des polypeptides, et le taux d'expression est déterminé au lieu de l'activité.

11. Le procédé selon la revendication 9 ou 10, où le composé d'essai est un ARN ou un peptide, ou un anticorps ou un inhibiteur de kinase.

12. Un kit pour le procédé de criblage d'un modulateur destiné à être utilisé pour le traitement d'une maladie du métabolisme des glucides comprenant :
a) un polypeptide GRK5,
b) un acide nucléique codant pour GRK5,
c) une lignée cellulaire de production d'insuline,
d) un composé de contrôle connu pour affecter la production d'insuline par liaison du polypeptide GRK5 ou de l'acide nucléique correspondant.


### SEQUENCE LISTING

<110> Max-Planck-Gesellschaft zur Förderung der Wissenschaften e.V.
<120> Kinases as targets for anti-diabetic therapy
<130> ULA-P03213WO-EP(II)
<160> 12
<170> PatentIn version 3.5
<210> 1
   <211> 23
   <212> DNA
   <213> Mus musculus
<400> 1
   cggcggcgac gatgtggttc ttt 23
<210> 2
   <211> 22
   <212> DNA
   <213> Mus musculus
<400> 2
   cggcgttgcc ctgtgccgag ta 22
<210> 3
   <211> 23
   <212> DNA
   <213> Mus musculus
<400> 3
   ctgacacggg caaggctgag att 23
<210> 4
   <211> 23
   <212> DNA
   <213> Mus musculus
<400> 4
   gcgccctgat acaacaccga gac 23
<210> 5
   <211> 22
   <212> DNA
   <213> Mus musculus
<400> 5
   gccgggtgct ggagactgag ga 22
<210> 6
   <211> 24
   <212> DNA
   <213> Mus musculus
<400> 6
   tggcggttct ggaggctgac ttct 24
<210> 7
   <211> 2637
   <212> DNA
   <213> Mus musculus
<400> 7
   cccgccccgc cccggctcgg gcggccggag gacccggagt ggaggctccc gagcccgtgg 60
   cggcggcgac gatgtggttc tttgcccggg acccggtccg ggacttcccg ttcgagctga 120
   gccctgagcc ccccgaaggc gggccgcccg ggccctggat cctgcaccga ggccgcaaaa 180
   aggccacagg cagcgcagtg tccatcttcg tgtatgatgt gaaaccggga gctgaagagc 240
   agacccaggt ggccaaagct gccttcaaac gcctcaaaac tctccgacac cccaacatcc 300
   tggcctatat cgatgggttg gagacagaaa agtgcctcca catcgtgaca gaggctgtga 360
   cccccctggg aacatacctc aaggcacgag cagaagcagg tggcctgaag gagcaggagc 420
   tgtcatgggg gttacaccag atcgtgaaag ccctcagctt cctggtcaac gactgcaacc 480
   tcatccacaa taatgtctgc atggccgctg tgtttgtgga cagggctggc gagtggaaac 540
   ttgggggtct ggactacatg tactcggcac agggcaacgg cgggggacca cccagcaagg 600
   ggatcccgga gctcgagcag tatgatcccc cggagctggc tgacagcagt agcagagcag 660
   tcagagagaa gtggtcagca gacatgtggc gcttgggctg cctcatctgg gaagttttca 720
   atgggtctct acctcgggca gctgccctgc gcaaccctgg gaagatcccc aaatccctgg 780
   tgacccatta ctgtgaactg gtgggagcta acccaaaagt acgtcccaac ccggcccgct 840
   tcctgcagaa ctgccgggca cccggtggct tcatgagcaa ccgctttgtt gagaccaacc 900
   tcttcctgga ggagattcag atcaaagagc cagctgagaa gcagaagttc ttccaagagc 960
   tgagcaagag tctagactca tttcccgaag atttctgtcg acacaaggtg ctgccccagc 1020
   tactgactgc ctttgagttt ggcaatgctg gggccgtggt cctcacacct ctcttcaagg 1080
   tgggaaaatc cctccgtgct gaagagtacc aggagaagat catccccgtg gtagttaaga 1140
   tgttctcatc caccgaccgg gccatgcgca tccgcctcct ccagcagatg gagcagttca 1200
   tccaatacct tgatgagcca acagtcaaca cgcagatttt cccccacgtc acacatggct 1260
   tcctggacac caaccccgcc atccgcgagc agacggtcaa gtccatgctg ctcttggccc 1320
   caaagctgaa tgaggccaat ctcaatgtgg aactgatgaa gcactttgca aggctacaag 1380
   ccaaggacga ccagggtcct atccgctgca acaccacggt ctgcttgggc aaaatcggct 1440
   cctatctcag tgctagtact agacacaggg tcctcacctc cgccttcagc agagccacta 1500
   aggacccatt tgcaccatcc cgggttgcgg gtgtcctggg ctttgctgcc acacacaatc 1560
   tctattcgat ggacgactgt gcccataaga tcctgcctgt gctctgtggc cttactgtgg 1620
   accctgagaa atctgtgcgg gaccaggcct ttaagaccat tcgaagcttc ctgtccaaat 1680
   tagagtctgt gtcagaggat cccacccagc tggcagaagt agagaaggat gtccatgcag 1740
   cgtccagtcc tggaacagga ggagctgcag ccagctgggc aggctgggct gtgactgggg 1800
   tatcctctct cacctccaag ctgatccgag cacaccccac gcctgtgccg tctgatacca 1860
   ctgtgcccca gagaccagtg ccagagggaa atcctgctcc agcccctgcc cttgcccaag 1920
   ctatccctgc aacctcaggg cactgggaga cacaggaaga caaggacact gcagaagaca 1980
   gcgccactgc tgacagatgg gacgatgagg actggggcag cttggagcag gaagctgaat 2040
   ccgtgttggc acagcaggat gactggagtg ccaagggcca aggaagccga gctggacaga 2100
   tcaaccaccc agaccacaaa tctctggaat cacattggag cagctgggaa gttgagggct 2160
   cctgggacca gggctggcag gaacccagct ctgtggagcc acctccagaa ggcactcggc 2220
   tagctagcga atataactgg ggtggtgcag agcccagtga caagggcgac ccctttgctg 2280
   ccctgtctgt tcgtcccagc gctcagccca ggccagaccc agactcctgg ggtgaagaca 2340
   actgggaagg cttggaggct gagagcagac aggtaaaggc agagctggcc cggaaaaagc 2400
   gagaggaaag gagaagagaa atggaagcca aacgggcaga gaaaaagacc accaaggggc 2460
   ccatgaagct gggagcccgg aagctggact gacaacccca cccccaagcc actgggcttc 2520
   caaccactgg agagcaggcc cggcggatgt atttattgta caaaccatgt gagcctggtc 2580
   agcaggtcag gcacatctag tgtacataat cagagccaca ataaattcta tttcaca 2637
<210> 8
   <211> 3182
   <212> DNA
   <213> Mus musculus
<400> 8
   gcggccgcca ggtcgccccg ggctggccga accccttgct gcccggaaac aaagtgcacc      60
   tttgtcagag acctggcgcg tgtgagcggc agacggcggg ccgcgcggaa ccccaggagc     120
   gcgccttcgc tcgggtctca gctcactcct gggcctccca cagcagcgtt ctgccgcctt     180
   ggcttgtgct ttccgctttc tcggatcttg gcgggacagg aaagggactc tgcgctccag     240
   gagtgggggt ttcgttttgg ctgagtcgtt tttgactcct gccggcgagg ctgggccgcc     300
   tgggctcagc gccataccgg cagcagtcct gctccacagt cccggtgcaa tagagccctc     360
   gccccgtcag acgcgggact acaattccca gcactcctcg cgttcagagc gcgcggtggg     420
   agttgcctcc gggcagacgc ctgcgcgctg cagcggctcg agcctggagt accacacccc     480
   cgggagggga acgaggggag gctgaagcat ccgaagcatt aaagcatccg agggagccgg     540
   aggggaggag aatggagtga cagagacgcg cggagggtgg ggggtggggg ggaaagtgtt     600
   gagggagggg ggagggggga cacagaggga ggaagaagcg gcggcggcgt ctcttcggtg     660
   cagaggggga aactccgcgg gctccgagaa agaataatgc ggtagcaggc aggctgcttg 720
   ctctggggtt tggcagcagc ggcggcagct cgagcagtgg cagcggcagc ggcatcaccc 780
   cagacgctga cagcacagcc ggccggctcc ctcgctgact gccgactgtc aatggagctg 840
   gaaaacatcg tggccaacac ggtcttgctg aaagcccggg aagggggtgg aggaaagcgc 900
   aaagggaaaa gcaagaagtg gaaggaaatc ctgaagtttc ctcacatcag ccagtgtgaa 960
   gacctccgaa ggaccataga cagagattac tacagtctat gtgacaagca accaattggg 1020
   agactgcttt ttcgacagtt ctgtgaaacc aggcctgggc tggagtgcta cattcagttc 1080
   ctggacttag tggcagaata tgaaattact ccagatgaaa accttggggc gaaggggaag 1140
   gaaataatga ccaagtacct cactccaaag tccccagtct tcattgccca agttggacag 1200
   gacctggtct cccagacaga gaagaagctc ctgcagagcc cctgcaaaga actcttctct 1260
   gcttgtgctc agtctgtcca tgactacttg aagggagacc ccttccacga gtacctggat 1320
   agcatgtatt ttgaccgttt tctgcagtgg aaatggttag aaagacaacc agtgaccaaa 1380
   aacactttcc ggcagtaccg agtgctgggc aaagggggct ttggagaggt ctgtgcctgc 1440
   caggttcggg ccactggtaa aatgtatgct tgtaaacgct tagagaagaa gaggatcaaa 1500
   aagaggaaag gcgaatccat ggcactcaac gaaaagcaga ttcttgagaa ggtcaacagc 1560
   cagtttgtgg tcaacctggc ctatgcctat gaaaccaaag atgcactatg cctggttctg 1620
   accattatga atggtggtga cctgaagttt cacatctaca atatggggaa tcctggcttt 1680
   gaggaagagc gagccttatt ttatgcagct gagatcctct gtggcctaga agacttacac 1740
   cgtgagaaca ctgtctatag agatctaaaa cccgaaaaca tcttgctgga tgattatggc 1800
   cacataagga tctcagacct cggactggcc gtgaagatcc ccgagggaga ccttatccgt 1860
   ggccgggtag gcactgttgg ctacatggcc ccagaagttc tgaacaacca gcgatatgga 1920
   ctgagccctg actactgggg cctgggctgc ctcatctatg agatgattga aggccagtca 1980
   ccatttcgag gtcgcaagga gaaggttaag cgggaagagg tggatcgccg ggtgctggag 2040
   actgaggaag tgtattcctc caagttctct gaagaggcca agtccatctg caacatgctg 2100
   ctcaccaaag actcgaagca gaggctgggc tgccaggagg agggggccgc cgaggtcaag 2160
   aggcaccctt tcttcaggaa catgaacttt aagcgcctgg aggctgggat gttggaccct 2220
   cccttcgttc cagatccccg ggctgtatac tgcaaggatg tgctggacat tgagcagttc 2280
   tccactgtga aaggtgtcaa cctggaccat acggacgatg atttttactc aaagttctct 2340
   acaggctctg tgccaattcc atggcaaaat gagatgatag aaacagaatg tttcaaggag 2400
   ctgaatgtgt tcggacctaa cggtaccctc tcaccagacc tgaacagaag tcagcctcca 2460
   gaaccgccaa agaaagggct gttccacaga ctcttcaggc gtcagcatca aagcaattcc 2520
   aagagttcac ctactcctaa gaccagttgt aaccaccgaa taaattcaaa ccacatcaat      2580
   tcaaactcca ctggaagcag ctagtttcgg ctctggcctt gaagtcaaaa gtggaaccag      2640
   ctcagagcct tctacttgga agcagaactt gtagccaggg gagcttccac tgtggctcag      2700
   tggccagcaa agctccagtg ggaactaaga taggagaccg ttcccccaat aacaaacctc      2760
   caagtttctc aaagaaattt ccactcaggt ctgttttcca aggtggcccc aagctggggt      2820
   ggactagaat tttccttgtt gaacattgca atagaaaccc aatgggatat gacagcttgc      2880
   acggatttta atagcatcct aactagaact gaattttgtc tttattattt ttaaaggaaa      2940
   gttttgtaaa tttctctatt gtctctgttt acattttgta tatttgtatt taagtgaaag      3000
   tcagactttg agggtgtata ttttctgtgc agccactgtt aagccatgtg ttttaagaca      3060
   ttttagattg gagggggggt tacaaaaatg tgactctaga cttccagagc ctcaaaagag      3120
   ataatgtttt tattaaatat agaaaatatc tcacttttta cctttaaaaa aaaaaaaaaa      3180
   aa                                                                      3182
<210> 9
   <211> 2287
   <212> DNA
   <213> Mus musculus
<400> 9
   gcccccttgg ctgtgactct ctcgctcggt gtcctagagg tccttagtgt ggactggcca      60
   cttgggtggc ccaaccttgc gtgccctgga gtaggcagag gtgaagaggg gctacgagtg     120
   gctccgccgg cagccttcag acatcggtga ccttgtgggc tcccacgcag agggtctgga     180
   gacatggcca gaaaggctct caagcttgct tcatggacca gcgtggctct tgctgcctcc     240
   ggtgtctacc tctacagtaa caactacttg gaccctaatg actttggcgc tgtcagggtg     300
   ggcagagctg ttgctacgac agctgtcatc agctatgact acctcacctc cctgaggagt     360
   gtcccatatg gctctgagga gtatttgcag cgtcgatccc aggtgcacct ccgctctgcc     420
   aggcgtctct ttgagctctg ctgtgccaac cggggcactt tcatcaaggt gggccagcac     480
   ctgggggcgc tggactacct gctgccagaa gagtacacca gcacactgaa ggtgttgcac     540
   agtcaagccc cacagagcag catgcaagag gtccggcagg tcatccgaga agacctgggc     600
   aaggagatcc acgatttgtt cctgagcttc gatgacaccc ctcttggggc agcctccctg     660
   gcccaggtcc acaaggcggt gttgcatgat ggtcggacag tggcagttaa ggtccagcac     720
   ccgaaggttc aggctcaaag ctctaaggac attctcctga tggaggtgct tgtcctggcc     780
   gtgaagcaac ttttcccaga ttttgaattc atgtggctgg tggatgaagc gaagaagaac     840
   ctgcctctcg agttggactt cctgaatgaa gggaggaacg ctgagaaagt ggcccacatg     900
   ctcaggcact ttgacttcct aaaggttccc cagatccact gggagctgtc taccaagagg     960
   gtgctcctga tggaatttgt agagggaggt caagtcaacg acagggccta catggagaag 1020
   aaccagatcg atgtgaatga gatctcctgc cacctgggca agatgtacag tgagatgatc 1080
   tttgtcaatg gcttcgtgca ctgtgacccc cacccaggca acgtactggt acggaagcgt 1140
   cctgacacgg gcaaggctga gattgtcctc ttagaccatg ggctttacca ggtgctcacg 1200
   gaggagttcc gcctggacta ctgccatctg tggcagtctc tgatctggac tgacatggac 1260
   gggctgaaac agtacagcca gcgcctggga gctgcagacc tctacccact gtttgcctgt 1320
   atgctgacag cccggtcctg ggactcagtc aaacagggca ttgggcaagc tccagtctct 1380
   gctactgagg actcagagat tcgcaataat gcagcctgct acctgcctga gatcagccag 1440
   ctccttaacc atgtgcctcg ccagatgctg ctcatcctga agactaatga tctactccgt 1500
   agcattgaga ccaccctggg cacgcgctcc agtgccagtt ccttcctcaa catgtctcgg 1560
   tgttgtatca gggcgctggc tgaacacaag aagagggatg ccggctcttt cttcagaagg 1620
   actcagatat ctttcagtga ggcctttagc ctgtggcaga tcaaccttca tgaacttctg 1680
   cttcgagtga gggccttgag gctagcttgc tgggtctcag ctctcctggg ctggctgact 1740
   cgggctccac acagaatgtg atggtctttc cctgcccctt cgtagtgtct ttccacacct 1800
   cattccttcc ttcacgctgg gacgacccac tgacccatgg ctgcctaggg ttggctgtgg 1860
   tcccagagtg gtcatccatg gcaccctcat gctctgccat tggagcccct gccttggggt 1920
   ggccttgtct tgaggtcctg gaatgtcctg gagagtgaga tgggataaag caacctctct 1980
   cccatctcct gtgtgtgcca ttgacttggt catccctact tttatgagga ctgtgagaat 2040
   ggaccaacca cctgtgtcac aggggcgtgt ttaacttgta gggaataagt agaaactcag 2100
   aacctgcaga gaacagactt tatatttttg ctgtatcact cccaaagttg tctcgcctca 2160
   gtgaggaaga ctgcatctga gagggaacga tgcaactgtg ggctcatgct gtcatggtga 2220
   caccttcagt gttatatgta ttgtatatat tgtttattgt aataaaccaa taaacagttt 2280
   caaggtt 2287
<210> 10
   <211> 806
   <212> PRT
   <213> Mus musculus
<400> 10
   Met Trp Phe Phe Ala Arg Asp Pro Val Arg Asp Phe Pro Phe Glu Leu
   1               5                   10                  15
   Ser Pro Glu Pro Pro Glu Gly Gly Pro Pro Gly Pro Trp Ile Leu His
               20                  25                  30
   Arg Gly Arg Lys Lys Ala Thr Gly Ser Ala Val Ser Ile Phe Val Tyr
           35                  40                  45
   Asp Val Lys Pro Gly Ala Glu Glu Gln Thr Gln Val Ala Lys Ala Ala
       50                  55                  60
   Phe Lys Arg Leu Lys Thr Leu Arg His Pro Asn Ile Leu Ala Tyr Ile
   65                  70                  75                  80
   Asp Gly Leu Glu Thr Glu Lys Cys Leu His Ile Val Thr Glu Ala Val
                   85                  90                  95
   Thr Pro Leu Gly Thr Tyr Leu Lys Ala Arg Ala Glu Ala Gly Gly Leu
               100                 105                 110
   Lys Glu Gln Glu Leu Ser Trp Gly Leu His Gln Ile Val Lys Ala Leu
           115                 120                 125
   Ser Phe Leu Val Asn Asp Cys Asn Leu Ile His Asn Asn Val Cys Met
       130                 135                 140
   Ala Ala Val Phe Val Asp Arg Ala Gly Glu Trp Lys Leu Gly Gly Leu
   145                 150                 155                 160
   Asp Tyr Met Tyr Ser Ala Gln Gly Asn Gly Gly Gly Pro Pro Ser Lys
                   165                 170                 175
   Gly Ile Pro Glu Leu Glu Gln Tyr Asp Pro Pro Glu Leu Ala Asp Ser
               180                 185                 190
   Ser Ser Arg Ala Val Arg Glu Lys Trp Ser Ala Asp Met Trp Arg Leu
           195                 200                 205
   Gly Cys Leu Ile Trp Glu Val Phe Asn Gly Ser Leu Pro Arg Ala Ala
       210                 215                 220
   Ala Leu Arg Asn Pro Gly Lys Ile Pro Lys Ser Leu Val Thr His Tyr
   225                 230                 235                 240
   Cys Glu Leu Val Gly Ala Asn Pro Lys Val Arg Pro Asn Pro Ala Arg
                   245                 250                 255
   Phe Leu Gln Asn Cys Arg Ala Pro Gly Gly Phe Met Ser Asn Arg Phe
               260                 265                 270
   Val Glu Thr Asn Leu Phe Leu Glu Glu Ile Gln Ile Lys Glu Pro Ala
           275                 280                 285
   Glu Lys Gln Lys Phe Phe Gln Glu Leu Ser Lys Ser Leu Asp Ser Phe
       290                 295                 300
   Pro Glu Asp Phe Cys Arg His Lys Val Leu Pro Gln Leu Leu Thr Ala
   305                 310                 315                 320
   Phe Glu Phe Gly Asn Ala Gly Ala Val Val Leu Thr Pro Leu Phe Lys
                   325                 330                 335
   Val Gly Lys Ser Leu Arg Ala Glu Glu Tyr Gln Glu Lys Ile Ile Pro
               340                 345                 350
   Val Val Val Lys Met Phe Ser Ser Thr Asp Arg Ala Met Arg Ile Arg
           355                 360                 365
   Leu Leu Gln Gln Met Glu Gln Phe Ile Gln Tyr Leu Asp Glu Pro Thr
       370                 375                 380
   Val Asn Thr Gln Ile Phe Pro His Val Thr His Gly Phe Leu Asp Thr
   385                 390                 395                 400
   Asn Pro Ala Ile Arg Glu Gln Thr Val Lys Ser Met Leu Leu Leu Ala
                   405                 410                 415
   Pro Lys Leu Asn Glu Ala Asn Leu Asn Val Glu Leu Met Lys His Phe
               420                 425                 430
   Ala Arg Leu Gln Ala Lys Asp Asp Gln Gly Pro Ile Arg Cys Asn Thr
           435                 440                 445
   Thr Val Cys Leu Gly Lys Ile Gly Ser Tyr Leu Ser Ala Ser Thr Arg
       450                 455                 460
   His Arg Val Leu Thr Ser Ala Phe Ser Arg Ala Thr Lys Asp Pro Phe
   465                 470                 475                 480
   Ala Pro Ser Arg Val Ala Gly Val Leu Gly Phe Ala Ala Thr His Asn
                   485                 490                 495
   Leu Tyr Ser Met Asp Asp Cys Ala His Lys Ile Leu Pro Val Leu Cys
               500                 505                 510
   Gly Leu Thr Val Asp Pro Glu Lys Ser Val Arg Asp Gln Ala Phe Lys
           515                 520                 525
   Thr Ile Arg Ser Phe Leu Ser Lys Leu Glu Ser Val Ser Glu Asp Pro
       530                 535                 540
   Thr Gln Leu Ala Glu Val Glu Lys Asp Val His Ala Ala Ser Ser Pro
   545 550 555 560
   Gly Thr Gly Gly Ala Ala Ala Ser Trp Ala Gly Trp Ala Val Thr Gly
   565 570 575
   Val Ser Ser Leu Thr Ser Lys Leu Ile Arg Ala His Pro Thr Pro Val
   580 585 590
   Pro Ser Asp Thr Thr Val Pro Gln Arg Pro Val Pro Glu Gly Asn Pro
   595 600 605
   Ala Pro Ala Pro Ala Leu Ala Gln Ala Ile Pro Ala Thr Ser Gly His
   610 615 620
   Trp Glu Thr Gln Glu Asp Lys Asp Thr Ala Glu Asp Ser Ala Thr Ala
   625 630 635 640
   Asp Arg Trp Asp Asp Glu Asp Trp Gly Ser Leu Glu Gln Glu Ala Glu
   645 650 655
   Ser Val Leu Ala Gln Gln Asp Asp Trp Ser Ala Lys Gly Gln Gly Ser
   660 665 670
   Arg Ala Gly Gln Ile Asn His Pro Asp His Lys Ser Leu Glu Ser His
   675 680 685
   Trp Ser Ser Trp Glu Val Glu Gly Ser Trp Asp Gln Gly Trp Gln Glu
   690 695 700
   Pro Ser Ser Val Glu Pro Pro Pro Glu Gly Thr Arg Leu Ala Ser Glu
   705 710 715 720
   Tyr Asn Trp Gly Gly Ala Glu Pro Ser Asp Lys Gly Asp Pro Phe Ala
   725 730 735
   Ala Leu Ser Val Arg Pro Ser Ala Gln Pro Arg Pro Asp Pro Asp Ser
   740 745 750
   Trp Gly Glu Asp Asn Trp Glu Gly Leu Glu Ala Glu Ser Arg Gln Val
   755 760 765
   Lys Ala Glu Leu Ala Arg Lys Lys Arg Glu Glu Arg Arg Arg Glu Met
   770 775 780
   Glu Ala Lys Arg Ala Glu Lys Lys Thr Thr Lys Gly Pro Met Lys Leu
   785 790 795 800
   Gly Ala Arg Lys Leu Asp
   805
<210> 11
   <211> 590
   <212> PRT
   <213> Mus musculus
<400> 11
   Met Glu Leu Glu Asn Ile Val Ala Asn Thr Val Leu Leu Lys Ala Arg
   1               5                   10                  15
   Glu Gly Gly Gly Gly Lys Arg Lys Gly Lys Ser Lys Lys Trp Lys Glu
               20                  25                  30
   Ile Leu Lys Phe Pro His Ile Ser Gln Cys Glu Asp Leu Arg Arg Thr
           35                  40                  45
   Ile Asp Arg Asp Tyr Tyr Ser Leu Cys Asp Lys Gln Pro Ile Gly Arg
       50                  55                  60
   Leu Leu Phe Arg Gln Phe Cys Glu Thr Arg Pro Gly Leu Glu Cys Tyr
   65                  70                  75                  80
   Ile Gln Phe Leu Asp Leu Val Ala Glu Tyr Glu Ile Thr Pro Asp Glu
                   85                  90                  95
   Asn Leu Gly Ala Lys Gly Lys Glu Ile Met Thr Lys Tyr Leu Thr Pro
               100                 105                 110
   Lys Ser Pro Val Phe Ile Ala Gln Val Gly Gln Asp Leu Val Ser Gln
           115                 120                 125
   Thr Glu Lys Lys Leu Leu Gln Ser Pro Cys Lys Glu Leu Phe Ser Ala
       130                 135                 140
   Cys Ala Gln Ser Val His Asp Tyr Leu Lys Gly Asp Pro Phe His Glu
   145                 150                 155                 160
   Tyr Leu Asp Ser Met Tyr Phe Asp Arg Phe Leu Gln Trp Lys Trp Leu
                   165                 170                 175
   Glu Arg Gln Pro Val Thr Lys Asn Thr Phe Arg Gln Tyr Arg Val Leu
               180                 185                 190
   Gly Lys Gly Gly Phe Gly Glu Val Cys Ala Cys Gln Val Arg Ala Thr
           195                 200                 205
   Gly Lys Met Tyr Ala Cys Lys Arg Leu Glu Lys Lys Arg Ile Lys Lys
       210                 215                 220
   Arg Lys Gly Glu Ser Met Ala Leu Asn Glu Lys Gln Ile Leu Glu Lys
   225                 230                 235                 240
   Val Asn Ser Gln Phe Val Val Asn Leu Ala Tyr Ala Tyr Glu Thr Lys
                   245                 250                 255
   Asp Ala Leu Cys Leu Val Leu Thr Ile Met Asn Gly Gly Asp Leu Lys
               260                 265                 270
   Phe His Ile Tyr Asn Met Gly Asn Pro Gly Phe Glu Glu Glu Arg Ala
           275                 280                 285
   Leu Phe Tyr Ala Ala Glu Ile Leu Cys Gly Leu Glu Asp Leu His Arg
       290                 295                 300
   Glu Asn Thr Val Tyr Arg Asp Leu Lys Pro Glu Asn Ile Leu Leu Asp
   305                 310                 315                 320
   Asp Tyr Gly His Ile Arg Ile Ser Asp Leu Gly Leu Ala Val Lys Ile
                   325                 330                 335
   Pro Glu Gly Asp Leu Ile Arg Gly Arg Val Gly Thr Val Gly Tyr Met
               340                 345                 350
   Ala Pro Glu Val Leu Asn Asn Gln Arg Tyr Gly Leu Ser Pro Asp Tyr
           355                 360                 365
   Trp Gly Leu Gly Cys Leu Ile Tyr Glu Met Ile Glu Gly Gln Ser Pro
       370                 375                 380
   Phe Arg Gly Arg Lys Glu Lys Val Lys Arg Glu Glu Val Asp Arg Arg
   385                 390                 395                 400
   Val Leu Glu Thr Glu Glu Val Tyr Ser Ser Lys Phe Ser Glu Glu Ala
                   405                 410                 415
   Lys Ser Ile Cys Asn Met Leu Leu Thr Lys Asp Ser Lys Gln Arg Leu
               420                 425                 430
   Gly Cys Gln Glu Glu Gly Ala Ala Glu Val Lys Arg His Pro Phe Phe
           435                 440                 445
   Arg Asn Met Asn Phe Lys Arg Leu Glu Ala Gly Met Leu Asp Pro Pro
       450                 455                 460
   Phe Val Pro Asp Pro Arg Ala Val Tyr Cys Lys Asp Val Leu Asp Ile
   465                 470                 475                 480
   Glu Gln Phe Ser Thr Val Lys Gly Val Asn Leu Asp His Thr Asp Asp
                   485                 490                 495
   Asp Phe Tyr Ser Lys Phe Ser Thr Gly Ser Val Pro Ile Pro Trp Gln
               500                 505                 510
   Asn Glu Met Ile Glu Thr Glu Cys Phe Lys Glu Leu Asn Val Phe Gly
           515                 520                 525
   Pro Asn Gly Thr Leu Ser Pro Asp Leu Asn Arg Ser Gln Pro Pro Glu
       530                 535                 540
   Pro Pro Lys Lys Gly Leu Phe His Arg Leu Phe Arg Arg Gln His Gln
   545                 550                 555                 560
   Ser Asn Ser Lys Ser Ser Pro Thr Pro Lys Thr Ser Cys Asn His Arg
                   565                 570                 575
   Ile Asn Ser Asn His Ile Asn Ser Asn Ser Thr Gly Ser Ser
               580                 585                 590
<210> 12
   <211> 551
   <212> PRT
   <213> Mus musculus
<400> 12
   Met Ala Arg Lys Ala Leu Lys Leu Ala Ser Trp Thr Ser Val Ala Leu
   1               5                   10                  15
   Ala Ala Ser Gly Val Tyr Leu Tyr Ser Asn Asn Tyr Leu Asp Pro Asn
               20                  25                  30
   Asp Phe Gly Ala Val Arg Val Gly Arg Ala Val Ala Thr Thr Ala Val
           35                  40                  45
   Ile Ser Tyr Asp Tyr Leu Thr Ser Leu Arg Ser Val Pro Tyr Gly Ser
       50                  55                  60
   Glu Glu Tyr Leu Gln Arg Arg Ser Gln Val His Leu Arg Ser Ala Arg
   65                  70                  75                  80
   Arg Leu Phe Glu Leu Cys Cys Ala Asn Arg Gly Thr Phe Ile Lys Val
   85 90 95
   Gly Gln His Leu Gly Ala Leu Asp Tyr Leu Leu Pro Glu Glu Tyr Thr
   100 105 110
   Ser Thr Leu Lys Val Leu His Ser Gln Ala Pro Gln Ser Ser Met Gln
   115 120 125
   Glu Val Arg Gln Val Ile Arg Glu Asp Leu Gly Lys Glu Ile His Asp
   130 135 140
   Leu Phe Leu Ser Phe Asp Asp Thr Pro Leu Gly Ala Ala Ser Leu Ala
   145 150 155 160
   Gln Val His Lys Ala Val Leu His Asp Gly Arg Thr Val Ala Val Lys
   165 170 175
   Val Gln His Pro Lys Val Gln Ala Gln Ser Ser Lys Asp Ile Leu Leu
   180 185 190
   Met Glu Val Leu Val Leu Ala Val Lys Gln Leu Phe Pro Asp Phe Glu
   195 200 205
   Phe Met Trp Leu Val Asp Glu Ala Lys Lys Asn Leu Pro Leu Glu Leu
   210 215 220
   Asp Phe Leu Asn Glu Gly Arg Asn Ala Glu Lys Val Ala His Met Leu
   225 230 235 240
   Arg His Phe Asp Phe Leu Lys Val Pro Gln Ile His Trp Glu Leu Ser
   245 250 255
   Thr Lys Arg Val Leu Leu Met Glu Phe Val Glu Gly Gly Gln Val Asn
   260 265 270
   Asp Arg Ala Tyr Met Glu Lys Asn Gln Ile Asp Val Asn Glu Ile Ser
   275 280 285
   Cys His Leu Gly Lys Met Tyr Ser Glu Met Ile Phe Val Asn Gly Phe
   290 295 300
   Val His Cys Asp Pro His Pro Gly Asn Val Leu Val Arg Lys Arg Pro
   305 310 315 320
   Asp Thr Gly Lys Ala Glu Ile Val Leu Leu Asp His Gly Leu Tyr Gln
   325 330 335
   Val Leu Thr Glu Glu Phe Arg Leu Asp Tyr Cys His Leu Trp Gln Ser
               340                 345                 350
   Leu Ile Trp Thr Asp Met Asp Gly Leu Lys Gln Tyr Ser Gln Arg Leu
           355                 360                 365
   Gly Ala Ala Asp Leu Tyr Pro Leu Phe Ala Cys Met Leu Thr Ala Arg
       370                 375                 380
   Ser Trp Asp Ser Val Lys Gln Gly Ile Gly Gln Ala Pro Val Ser Ala
   385                 390                 395                 400
   Thr Glu Asp Ser Glu Ile Arg Asn Asn Ala Ala Cys Tyr Leu Pro Glu
                   405                 410                 415
   Ile Ser Gln Leu Leu Asn His Val Pro Arg Gln Met Leu Leu Ile Leu
               420                 425                 430
   Lys Thr Asn Asp Leu Leu Arg Ser Ile Glu Thr Thr Leu Gly Thr Arg
           435                 440                 445
   Ser Ser Ala Ser Ser Phe Leu Asn Met Ser Arg Cys Cys Ile Arg Ala
       450                 455                 460
   Leu Ala Glu His Lys Lys Arg Asp Ala Gly Ser Phe Phe Arg Arg Thr
   465                 470                 475                 480
   Gln Ile Ser Phe Ser Glu Ala Phe Ser Leu Trp Gln Ile Asn Leu His
                   485                 490                 495
   Glu Leu Leu Leu Arg Val Arg Ala Leu Arg Leu Ala Cys Trp Val Ser
               500                 505                 510
   Ala Leu Leu Gly Trp Leu Thr Arg Ala Pro His Arg Met Arg Arg Glu
           515                 520                 525
   Met Glu Ala Lys Arg Ala Glu Lys Lys Thr Thr Lys Gly Pro Met Lys
       530                 535                 540
   Leu Gly Ala Arg Lys Leu Asp
   545                 550